# EUROPEAN PATENT APPLICATION

(11) **EP 3 964 579 A1**
(43) Date of publication of application: **09.03.2022**
(21) Application number: 20798490.7
(22) Date of filing: 29.04.2020
(51) Int. Cl.: C12N 15/11, A61P 7/00

(54) **METHOD FOR PREDICTING EFFECTIVENESS OF TREATMENT OF HEMOGLOBINOPATHY**

(30) Priority: 30.04.2019 WO PCT/CN2019/085116
(71) Applicant: Edigene Inc., Beijing 102206 (CN)
(72) Inventor: FANG, Riguo, Beijing 102206 (CN); YU, Lingling, Beijing 102206 (CN); YANG, Huihui, Beijing 102206 (CN)
(74) Representative: Lavoix
(86) International application number: PCT/CN2020/087766
(87) International publication number: WO 2020/221291

(57) **Abstract**

The present invention relates to a method for treating hemoglobinopathy in an individual, comprising: (a) an evaluation step: the evaluation step comprises evaluating the ability of a first population of modified CD34-positive hematopoietic stem cells/progenitor cells to produce a desired level of γ-globin or fetal hemoglobin after differentiation, the modified CD34-positive HSPCs of the first population being derived from the individual and being modified to reduce BCL11A function; and (b) a treatment step: the treatment step comprises administering to the individual a second population of modified CD34-positive HSPCs, the modified CD34-positive HSPCs being derived from the individual and being modified to reduce BCL11A function. At the same time, the invention also relates to a method for treating hemoglobinopathy in individuals, a method for selecting individuals suffering from hemoglobinopathy for treatment using the modified CD34-positive HSPCs of the second population, and a method for determining whether an individual suffering from hemoglobinopathy is suitable or unsuitable for treatment using the second population of modified CD34-positive HSPCs derived from the individual and modified to reduce BCL11A function.

SEQUENCE LISTING

<110>  Edigene Inc.

<120>  Method for Predicting Effectiveness of Treatment of Hemoglobinopathy

<130>  FD00222PCT-PD01017

<150>  PCT/CN2019/085116
<151>  2019-04-30

<160>  27

<170>  PatentIn version 3.5

<210>  1
<211>  4101
<212>  DNA
<213>  Artificial Sequence

<220>
<223>  Cas9 mRNA coding gene

<400>  1
gacaagaagt acagcatcgg cctggacatc ggcaccaact ctgtgggctg ggccgtgatc      60

accgacgagt acaaggtgcc cagcaagaaa ttcaaggtgc tgggcaacac cgaccggcac     120

agcatcaaga agaacctgat cggagccctg ctgttcgaca gcggcgaaac agccgaggcc     180

acccggctga agagaaccgc cagaagaaga tacaccagac ggaagaaccg gatctgctat     240

ctgcaagaga tcttcagcaa cgagatggcc aaggtggacg acagcttctt ccacagactg     300

gaagagtcct tcctggtgga agaggataag aagcacgagc ggcaccccat cttcggcaac     360

atcgtggacg aggtggccta ccacgagaag taccccacca tctaccacct gagaaagaaa     420

ctggtggaca gcaccgacaa ggccgacctg cggctgatct atctggccct ggcccacatg     480

atcaagttcc ggggccactt cctgatcgag ggcgacctga accccgacaa cagcgacgtg     540

gacaagctgt tcatccagct ggtgcagacc tacaaccagc tgttcgagga aaaccccatc     600

aacgccagcg gcgtggacgc caaggccatc ctgtctgcca gactgagcaa gagcagacgg     660

ctggaaaatc tgatcgccca gctgcccggc gagaagaaga atggcctgtt cggcaacctg     720

attgccctga gcctgggcct gacccccaac ttcaagagca acttcgacct ggccgaggat     780

gccaaactgc agctgagcaa ggacacctac gacgacgacc tggacaacct gctggcccag     840

atcggcgacc agtacgccga cctgtttctg gccgccaaga acctgtccga cgccatcctg     900

ctgagcgaca tcctgagagt gaacaccgag atcaccaagg cccccctgag cgcctctatg     960

atcaagagat acgacgagca ccaccaggac ctgaccctgc tgaaagctct cgtgcggcag    1020

cagctgcctg agaagtacaa agagattttc ttcgaccaga gcaagaacgg ctacgccggc    1080

tacattgacg gcggagccag ccaggaagag ttctacaagt tcatcaagcc catcctggaa    1140

aagatggacg gcaccgagga actgctcgtg aagctgaaca gagaggacct gctgcggaag    1200

cagcggacct tcgacaacgg cagcatcccc caccagatcc acctgggaga gctgcacgcc 1260

attctgcggc ggcaggaaga tttttaccca ttcctgaagg acaaccggga aaagatcgag 1320

aagatcctga ccttccgcat cccctactac gtgggccctc tggccagggg aaacagcaga 1380

ttcgcctgga tgaccagaaa gagcgaggaa accatcaccc cctggaactt cgaggaagtg 1440

gtggacaagg gcgcttccgc ccagagcttc atcgagcgga tgaccaactt cgataagaac 1500

ctgcccaacg agaaggtgct gcccaagcac agcctgctgt acgagtactt caccgtgtat 1560

aacgagctga ccaaagtgaa atacgtgacc gagggaatga gaaagcccgc cttcctgagc 1620

ggcgagcaga aaaaggccat cgtggacctg ctgttcaaga ccaaccggaa agtgaccgtg 1680

aagcagctga aagaggacta cttcaagaaa atcgagtgct tcgactccgt ggaaatctcc 1740

ggcgtggaag atcggttcaa cgcctccctg ggcacatacc acgatctgct gaaaattatc 1800

aaggacaagg acttcctgga caatgaggaa aacgaggaca ttctggaaga tatcgtgctg 1860

accctgacac tgtttgagga cagagagatg atcgaggaac ggctgaaaac ctatgcccac 1920

ctgttcgacg acaaagtgat gaagcagctg aagcggcgga gatacaccgg ctggggcagg 1980

ctgagccgga agctgatcaa cggcatccgg gacaagcagt ccggcaagac aatcctggat 2040

ttcctgaagt ccgacggctt cgccaacaga aacttcatgc agctgatcca cgacgacagc 2100

ctgaccttta aagaggacat ccagaaagcc caggtgtccg gccagggcga tagcctgcac 2160

gagcacattg ccaatctggc cggcagcccc gccattaaga agggcatcct gcagacagtg 2220

aaggtggtgg acgagctcgt gaaagtgatg ggccggcaca agcccgagaa catcgtgatc 2280

gaaatggcca gagagaacca gaccacccag aagggacaga agaacagccg cgagagaatg 2340

aagcggatcg aagagggcat caaagagctg ggcagccaga tcctgaaaga acaccccgtg 2400

gaaaacaccc agctgcagaa cgagaagctg tacctgtact acctgcagaa tgggcgggat 2460

atgtacgtgg accaggaact ggacatcaac cggctgtccg actacgatgt ggaccatatc 2520

gtgcctcaga gctttctgaa ggacgactcc atcgacaaca aggtgctgac cagaagcgac 2580

aagaaccggg gcaagagcga caacgtgccc tccgaagagg tcgtgaagaa gatgaagaac 2640

tactggcggc agctgctgaa cgccaagctg attacccaga gaaagttcga caatctgacc 2700

aaggccgaga gaggcggcct gagcgaactg gataaggccg gcttcatcaa gagacagctg 2760

gtggaaaccc ggcagatcac aaagcacgtg gcacagatcc tggactcccg gatgaacact 2820

aagtacgacg agaatgacaa gctgatccgg gaagtgaaag tgatcaccct gaagtccaag 2880

ctggtgtccg atttccggaa ggatttccag ttttacaaag tgcgcgagat caacaactac 2940

caccacgccc acgacgccta cctgaacgcc gtcgtgggaa ccgccctgat caaaaagtac 3000

cctaagctgg aaagcgagtt cgtgtacggc gactacaagg tgtacgacgt gcggaagatg 3060

atcgccaaga gcgagcagga aatcggcaag gctaccgcca agtacttctt ctacagcaac 3120

atcatgaact ttttcaagac cgagattacc ctggccaacg gcgagatccg gaagcggcct 3180

ctgatcgaga caaacggcga aaccggggag atcgtgtggg ataagggccg ggattttgcc 3240

accgtgcgga aagtgctgag catgccccaa gtgaatatcg tgaaaaagac cgaggtgcag 3300

acaggcggct tcagcaaaga gtctatcctg cccaagagga acagcgataa gctgatcgcc 3360

agaaagaagg actgggaccc taagaagtac ggcggcttcg acagccccac cgtggcctat 3420

tctgtgctgg tggtggccaa agtggaaaag ggcaagtcca agaaactgaa gagtgtgaaa 3480

gagctgctgg ggatcaccat catggaaaga agcagcttcg agaagaatcc catcgacttt 3540

ctggaagcca agggctacaa agaagtgaaa aaggacctga tcatcaagct gcctaagtac 3600

tccctgttcg agctggaaaa cggccggaag agaatgctgg cctctgccgg cgaactgcag 3660

aagggaaacg aactggccct gccctccaaa tatgtgaact tcctgtacct ggccagccac 3720

tatgagaagc tgaagggctc ccccgaggat aatgagcaga aacagctgtt tgtggaacag 3780

cacaagcact acctggacga gatcatcgag cagatcagcg agttctccaa gagagtgatc 3840

ctggccgacg ctaatctgga caaagtgctg tccgcctaca acaagcaccg ggataagccc 3900

atcagagagc aggccgagaa tatcatccac ctgtttaccc tgaccaatct gggagcccct 3960

gccgccttca agtactttga caccaccatc gaccggaaga ggtacaccag caccaaagag 4020

gtgctggacg ccaccctgat ccaccagagc atcaccggcc tgtacgagac acggatcgac 4080

ctgtctcagc tgggaggcga c 4101

<210> 2
<211> 150
<212> DNA
<213> Artificial Sequence

<220>
<223> 150bp sequence at 58K site of BCL11A gene

<400> 2
ctgccagtcc tcttctaccc cacccacgcc cccaccctaa tcagaggcca aacccttcct 60

ggagcctgtg ataaaagcaa ctgttagctt gcactagact agcttcaaag ttgtattgac 120

cctggtgtgt tatgtctaag agtagatgcc 150

<210> 3
<211> 20
<212> DNA
<213> Artificial Sequence

<220>
<223> sgRNA encoding gene

<400> 3
cacaggctcc aggaagggtt 20

<210>  4
<211>  20
<212>  DNA
<213>  Artificial Sequence

<220>
<223>  sgRNA encoding sequence

<400>  4
atcagaggcc aaacccttcc                                                    20


<210>  5
<211>  20
<212>  DNA
<213>  Artificial Sequence

<220>
<223>  sgRNA encoding sequence

<400>  5
ctaacagttg cttttatcac                                                    20


<210>  6
<211>  20
<212>  DNA
<213>  Artificial Sequence

<220>
<223>  sgRNA encoding sequence

<400>  6
ttgcttttat cacaggctcc                                                    20


<210>  7
<211>  20
<212>  DNA
<213>  Artificial Sequence

<220>
<223>  sgRNA encoding sequence

<400>  7
ttttatcaca ggctccagga                                                    20


<210>  8
<211>  20
<212>  DNA
<213>  Artificial Sequence

<220>
<223>  sgRNA encoding sequence

<400>  8
tttatcacag gctccaggaa                                                    20


<210>  9

<211>  20
<212>  DNA
<213>  Artificial Sequence

<220>
<223>  sgRNA encoding sequence

<400>  9
tgggtggggt agaagaggac                                                    20


<210>  10
<211>  20
<212>  DNA
<213>  Artificial Sequence

<220>
<223>  sgRNA encoding sequence

<400>  10
gggcgtgggt ggggtagaag                                                    20


<210>  11
<211>  20
<212>  DNA
<213>  Artificial Sequence

<220>
<223>  sgRNA encoding sequence

<400>  11
ttagggtggg ggcgtgggtg                                                    20


<210>  12
<211>  20
<212>  DNA
<213>  Artificial Sequence

<220>
<223>  sgRNA encoding sequence

<400>  12
attagggtgg gggcgtgggt                                                    20


<210>  13
<211>  20
<212>  DNA
<213>  Artificial Sequence

<220>
<223>  sgRNA encoding sequence

<400>  13
gattagggtg ggggcgtggg                                                    20


<210>  14
<211>  20
<212>  DNA
<213>  Artificial Sequence

<220>
<223>  sgRNA encoding sequence

<400>  14
tctgattagg gtgggggcgt                                                   20


<210>  15
<211>  20
<212>  DNA
<213>  Artificial Sequence

<220>
<223>  sgRNA encoding sequence

<400>  15
ctctgattag ggtgggggcg                                                   20


<210>  16
<211>  20
<212>  DNA
<213>  Artificial Sequence

<220>
<223>  sgRNA encoding sequence

<400>  16
cacgccccca ccctaatcag                                                   20


<210>  17
<211>  20
<212>  DNA
<213>  Artificial Sequence

<220>
<223>  sgRNA encoding sequence

<400>  17
ttggcctctg attagggtgg                                                   20


<210>  18
<211>  20
<212>  DNA
<213>  Artificial Sequence

<220>
<223>  sgRNA encoding sequence

<400>  18
tttggcctct gattagggtg                                                   20


<210>  19
<211>  20
<212>  DNA
<213>  Artificial Sequence

<220>
<223>  sgRNA encoding sequence

<400>  19
gtttggcctc tgattagggt                                                   20


<210>  20
<211>  20
<212>  DNA
<213>  Artificial Sequence

<220>
<223>  sgRNA encoding sequence

<400>  20
ggtttggcct ctgattaggg                                                   20


<210>  21
<211>  20
<212>  DNA
<213>  Artificial Sequence

<220>
<223>  sgRNA encoding sequence

<400>  21
aagggtttgg cctctgatta                                                   20


<210>  22
<211>  20
<212>  DNA
<213>  Artificial Sequence

<220>
<223>  sgRNA encoding sequence

<400>  22
gaagggtttg gcctctgatt                                                   20


<210>  23
<211>  20
<212>  DNA
<213>  Artificial Sequence

<220>
<223>  sgRNA encoding sequence

<400>  23
actcttagac ataacacacc                                                   20


<210>  24
<211>  20
<212>  DNA
<213>  Artificial Sequence

<220>
<223>  sgRNA encoding sequence

<400>  24
cttcaaagtt gtattgaccc                                                   20

<210>  25
<211>  20
<212>  DNA
<213>  Artificial Sequence

<220>
<223>  sgRNA encoding sequence

<400>  25
ctcttagaca taacacacca                                                   20


<210>  26
<211>  24
<212>  DNA
<213>  Artificial Sequence

<220>
<223>  Forward sequencing primer

<400>  26
cacctcagca gaaacaaagt tatc                                              24


<210>  27
<211>  20
<212>  DNA
<213>  Artificial Sequence

<220>
<223>  Reverse sequencing primer

<400>  27
gggaagctcc aaactctcaa                                                   20

## Description

### CROSS REFERENCE TO RELATED APPLICATIONS

This application claims priority benefit of PCT application PCT/CN2019/085116 filed on April 30, 2019, which is incorporated herein by reference in its entirety.

### SUBMISSION OF SEQUENCE LISTING ON ASCII TEXT FILE

The content of the following submission on ASCII text file is incorporated herein by reference in its entirety: a computer readable form (CRF) of the Sequence Listing (file name: FD00222PCT-PD01017-Sequence listing_ST25, date recorded: April 22, 2020, size: 11 KB).

### TECHNICAL FIELD

This application relates to an accompanying prediction method for predicting the effectiveness of gene editing technology in treating hemoglobinopathy. In this prediction method, the CD34-positive hematopoietic stem cells of a patient are isolated prior to treating the disease, gene editing technology is used to destroy the BCL11A erythroid enhancer sites, and the effectiveness of treatment of hemoglobinopathy through gene-edited BCL11A erythroid enhancer is predicted in advance by evaluating the degree of up-regulation of γ-globin and fetal hemoglobin expression, thereby facilitating the treatment of the disease.

### BACKGROUND ART

Hemoglobinopathy is a group of inherited blood diseases caused by abnormal hemoglobin molecular structure or abnormal synthesis of globin peptide chain. The two main disease types in hemoglobinopathy are β-thalassemia and sickle cell anemia. The pathogenesis of β-thalassemia is due to gene mutations in the β-globin peptide chain; most patients have point mutations, and a few have large fragment gene deletions. Gene deletion and certain point mutations may cause the synthesis of a part of β-globin peptide chain to be completely inhibited, and this type is called β⁰ thalassemia; a few point mutations partially inhibit the synthesis of β chain, but the synthesis of a part of the peptide chain is still retained, this type is called β⁺ thalassemia, and different combinations may have different clinical symptoms. Due to the lack or decrease of β-globin expression, the excess α chains in the blood will be deposited in the erythrocytes to form inclusion bodies attaching to the surface of the erythrocyte membrane and changing the characteristics of the erythrocyte membrane, and the cells become stiff to result in a decrease in deformability, presenting the manifestations of chronic hemolytic anemia, thereby further changing the composition of bone marrow. Sickle cell anemia, similar to β-thalassemia, is an autosomal recessive inherited disease; the difference is that this anemia has a single mutation site, and is caused by a single base mutation of β-globin, i.e., the codon 6 of the normal β gene is mutated from GAG (encoding glutamic acid) to GTG (valine). In the mutant homozygous state, normal α-globin and abnormal β-globin form a tetramer complex called HbS, the tetramer has half the capacity of carrying oxygen as normal hemoglobin, and aggregates into polymers in the deoxygenated state; since the formed polymers are arranged in parallel with the membrane and in close contact with the cell membrane, when the number of polymers reaches a certain level, the cell membrane changes from a normal concave shape to a sickle shape. Sickle-shaped erythrocytes have poor deformability and are easily broken and hemolyzed, thereby causing blood vessel blockage, injury, and necrosis, etc. (D. Rund, et al. New England Journal of Medicine. 2005, 718-739).

Currently the standard therapeutic methods for β-thalassemia and sickle cell anemia include long-term high-dose blood transfusions accompanied by iron removal therapy with de-iron agents and the therapeutic technology of transplantation of allogeneic hematopoietic stem cells. However, the long-term high-dose blood transfusions accompanied by iron removal therapy with de-iron agents lead to iron overload, and one of the main causes of death in children with thalassemia is the organ damage caused by the deposition of large amounts of iron in vital organs of the patients, such as spleen, liver, heart and kidneys. Although transplantation of allogeneic hematopoietic stem cells may eradicate β-thalassemia and sickle cell anemia, due to death caused by the low proportion of being fully compatible for HLA matching and GVHD (graft-versus-host-disease) and immunological rejection after transplantation, the current therapeutic technology is difficult to meet the huge needs of the patients to be treated. In order to solve the problem of allogeneic hematopoietic stem cell therapeutic technology, transgene therapy and gene editing therapy based on genetically modified autologous hematopoietic stem cells have emerged, wherein the therapeutic solution of gene editing therapy is: to edit the patient's self-derived hematopoietic stem cells by gene editing tools, such as CRISPR/Cas9, zinc finer nulease (ZFN) and TALEN, etc., increasing the expression of fetal hemoglobin (HbF), then returning the genetically modified self-derived hematopoietic stem cells to the patient to restore the total hemoglobin to a normal level, thereby achieving the purpose of treating the disease.

The main mechanism of gene editing therapy for treating β-thalassemia and sickle cell anemia is to use a biological phenomenon naturally present in the human body, i.e., hemoglobin switching. Hemoglobin of an adult (HbA) is a tetrameric complex consisting of two α-globins and two β -globins, but as for a fetus in mother's body and a baby within 120 days after birth, the hemoglobin in a human body is HbF, i.e., a tetramer complex consisting of two α-globins and two γ-globins, which is characterized by strong oxygen carrying capacity and weak oxygen releasing capacity, thereby being beneficial for a fetus to obtain nutrients from mother's body. However, 120 days after the birth of the fetus, the erythrocytes in a human body switch from HbF to HbA, and begin to express normal hemoglobin HbA (Marina et al, Molecular Therapy. 2017; Megan D, et al. Blood. 2015). There is a class of patients in the clinic, although they carry mutations relating to β -thalassemia or sickle cell anemia, they have mild or no clinical symptoms; since the genes inhibiting HbF expression also carry mutations that reduce the expression of these genes, eventually the patient has hereditary persistence of fetal hemoglobin (HPFH), which compensates for the decrease of the overall hemoglobin level in the erythrocytes caused by the lack or decrease of β -globin, thereby making the overall hemoglobin close to or return to the normal level (Vinjamur DS, et al. Br. J. Haematol. 2018). Therefore, how to find a suitable gene target site and increase the expression of fetal hemoglobin in erythrocytes by gene editing has become a brand-new therapeutic strategy. However, the expression of HbF and HbA in a human body is in a relatively stable state of dynamic equilibrium, and the regulation mechanism is very complicated. Studies have shown that there is an LCR (locus control region) of about 16 kb at 40-60 kb upstream of β-globin, and this region comprises 5 DNase-hypersensitive sites, which participate in the regulation of expression of HbF and HbA. In addition, some key transcription factors involved in hematopoietic system development and erythrocyte differentiation and maturation are tightly bound in this region, including GATA-1, BCL11A, FOG1, NuRD, LRF, MYB, KLF1, TAL1, E2A, LMO2, and LDB1, etc., and they cooperatively regulate the expression of HbF and HbA (G. Lettre, et al. PNAS. 2008, 11869-11874; Marina et al, Molecular Therapy. 2017; Megan D, et al. Blood. 2015). At present, the suitable target found is the position of the BCL11A erythroid enhancer (+58), this region is modified by gene editing to down-regulate the expression of BCL11A gene, thereby relieving the inhibition of the expression of γ-globin and HbF by BCL11A gene and increasing the expression of γ-globin and HbF, without affecting the differentiation and development of other lineages; and some clinically asymptomatic patients also carry mutations at this site that cause HPFH (U. Manuela, et al. PNAS. 2008; P. Liu, et al. Nature Immunology. 2003; D.E. Bauer. Science. 2013; V.G. Sankaran, et al. Science. 2008; Matthew C., et al. Nature. 2015).

Although clinical trials have been carried out for the BCL11A erythroid enhancer sites, i.e., the clinical Phasel/2 trials for treating β-thalassemia and sickle cell anemia through transplantation of autologous hematopoietic stem cells developed by gene editing technology (Clinicaltrials.gov numbers: NCT03655678, NCT03745287, and NCT03432364), but due to individual differences between patients, the degree of regulation of BCL11A gene on HbF is different in different patients. For example, if the BCL11A gene is already in a state of very low expression in a certain type of patients, indicating that the inhibition of expression of HbF by BCL11A gene is relieved, then it may fail to treat a patient by editing the BCL11A erythroid enhancer to increase HbF; and if the patient has undergone chemotherapy to clear the bone marrow and immune system, this will cause unpredictable potential harm. Moreover, the mechanism of regulating the expression of γ-globin and HbF fetal hemoglobin is very complicated, and it is difficult to predict the effectiveness of a therapeutic method in advance by detecting the expression of a certain gene or several genes. Therefore, how to predict in advance the degree and potential of the up-regulation of the expression of γ-globin and HbF by the BCL11A gene is a key issue that needs to be solved urgently in gene-edited autologous hematopoietic stem cell therapy.

### CONTENTS OF THE INVENTION

This application utilizes the strategy of pre-evaluating the degree of regulation of the BCL11A gene on HbF, and develops an accompanying diagnostic method for predicting the effectiveness of gene editing technology in the treatment of hemoglobinopathy, predicting in advance the degree of increasing the expression of γ-globin and HbF by gene editing of BCL11A erythroid enhancer sites, reducing the risk of failure or reduced effectiveness of the therapy due to the low expression or low function of BCL11A in the patient's cells, thereby facilitating to grade the patients for diagnosis and treatment during the clinical treatment process. A patient has high expression of BCL11A and/or a patient in which BCL11A plays a major role in regulating γ-globin and HbF is preferentially selected for performing the treatment, a novel therapeutic solution of accompanying diagnosis plus gene-edited autologous hematopoietic stem cells is developed to fill a gap in the existing technology, facilitating the quick development of the novel therapeutic strategy of treating hemoglobinopathy with gene-edited autologous hematopoietic stem cells, thereby meeting the needs of clinical applications.

This application proves for the first time through experiments that, as for the hematopoietic stem cells derived from mobilized peripheral blood of different donors, after the same sites of the BCL11A erythroid enhancer are efficiently gene-edited, the γ-globin mRNA level and HbF protein level after erythroid differentiation are increased differently, indicating that individual differences affect the inhibitory potential and degree of BCL11A on the expression of γ-globin and HbF protein. In addition, as for the hematopoietic stem cells derived from mobilized peripheral blood of the same donor, after the same sites of the BCL11A erythroid enhancer are efficiently gene-edited in different batches of experiments, the γ-globin mRNA level and HbF protein level after erythroid differentiation are increased stably and similarly. Further experimental results show that, as for a donor with higher expression of γ-globin and HbF protein in the same batch, the expression of γ-globin and HbF protein in another batch of experiments is also higher, and vice versa. Finally, this application proposes for the first time a novel therapeutic solution and strategy for treating hemoglobinopathy with accompanying diagnosis + gene-editing of BCL11A erythroid enhancer sites of autologous hematopoietic stem cells.

In one aspect, the present application provides a method for treating hemoglobinopathy in an individual, which comprises:
a) an evaluation step comprising: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV (evaluation) cells"); and
b) a treatment step comprising: administering a second population of the modified CD34-positive HSPCs to the individual, wherein the modified CD34-positive HSPCs are derived from the individual and are modified to reduce BCL11A function ("modified TR (treatment) cells").

In one aspect, the present application provides a method for enhancing the expression of fetal hemoglobin in an individual, which comprises:
a) an evaluation step comprising: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells"); and
b) a treatment step comprising: administering a second population of the modified CD34-positive HSPCs to the individual, wherein the modified CD34-positive HSPCs are derived from the individual and are modified to reduce BCL11A function ("modified TR cells").

In one aspect, this application provides a method for evaluating the function of hemoglobin in an individual, which comprises:
a) an evaluation step comprising: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells"); and
b) a treatment step comprising: administering a second population of the modified CD34-positive HSPCs to the individual, wherein the modified CD34-positive HSPCs are derived from the individual and are modified to reduce BCL11A function ("modified TR cells"). In one aspect, the present application provides a method for treating hemoglobinopathy in an individual, which comprises a treatment step comprising: administering a second population of the modified CD34-positive HSPCs to the individual, wherein the modified CD34-positive HSPCs are derived from the individual and are modified to reduce BCL11A function ("modified TR cells"),
wherein the individual is selected for treatment based on the functional evaluation from an evaluation step which comprises: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells").

In one aspect, the present application provides a method for selecting an individual suffering from hemoglobinopathy for treatment with a second population of the modified CD34-positive HSPCs which are derived from the individual and are modified to reduce BCL11A function ("modified TR cells"), wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), where if the first population of the modified CD34-positive HSPCs produce a desired level of γ-globin or fetal hemoglobin (HbF), the individual is selected for treatment.

In one aspect, the application provides a method for determining whether an individual suffering from hemoglobinopathy is suitable for treating with a second population of the modified CD34-positive HSPCs ("modified TR cells") derived from the individual and modified to reduce BCL11A function, wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), and where if the first population of the modified CD34-positive HSPCs produce a desired level of γ-globin or fetal hemoglobin (HbF), the individual is suitable for treatment.

In one aspect, the application provides a method for determining whether an individual suffering from hemoglobinopathy is unsuitable for treating with a second population of the modified CD34-positive HSPCs ("modified TR cells") derived from the individual and modified to reduce BCL11A function, wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), and wherein if the first population of the modified CD34-positive HSPCs do not produce a desired level of γ-globin or fetal hemoglobin (HbF), the individual is not suitable for treating with the modified TR cells.

In the above embodiment, evaluating the ability of a first population of the modified CD34-positive HSPCs to produce a desired level of γ-globin or fetal hemoglobin (HbF) after differentiation refers to: evaluating whether the level of γ-globin or fetal hemoglobin (HbF) produced by the modified CD34-positive HSPCs with reduced function of BCL11A after differentiation is increased by at least about 12%, 13%, 14%, 15%, 16%, 17%, 18%, 19%, 20%, 21%, 22%, 23%, 24%, 25%, 26%, 27%, 28%, 29%, 30%, 31% , 32%, 33%, 34%, 35%, 36%, 37%, 38%, 39%, 40%, 50%, 55%, 60%, 65%, 70%, 75%, 80%, 85% %, 90%, 95%, 100%, or 120% than the level of γ-globin or fetal hemoglobin (HbF) produced by the unmodified CD34-positive HSPCs after differentiation.

If the level of γ-globin or fetal hemoglobin (HbF) produced by a first population of the modified CD34-positive HSPCs after differentiation is increased by at least about 12%, 13%, 14%, 15%, 16%, 17%, 18%, 19%, 20%, 21%, 22%, 23%, 24%, 25%, 26%, 27%, 28%, 29%, 30%, 31% , 32%, 33%, 34%, 35%, 36%, 37%, 38%, 39%, 40%, 50%, 55%, 60%, 65%, 70%, 75%, 80%, 85% %, 90%, 95%, 100%, or 120% as compared with the level of γ-globin or fetal hemoglobin (HbF) produced by the unmodified CD34-positive HSPCs after differentiation, the individual is suitable for treatment with the modified TR cells.

In some embodiments of the above method, the level of γ-globin or fetal hemoglobin produced by the modified CD34-positive HSPCs after differentiation is 150%, 200%, 250%, 300%, 350%, 400%, 450%, 500%, 550%, 600%, or higher of the level of γ-globin or fetal hemoglobin produced by the unmodified CD34-positive HSPCs after differentiation.

In some embodiments of the above method, the fetal hemoglobin produced by the modified CD34-positive HSPCs after differentiation is more than 6.5 g/dL, 7.0 g/dL, 7.5 g/dL, 8.0 g/dL, 8.5 g/dL, 9.0 g/dL, 9.5g/dL, 10g/dL, 10.5g/dL, or 11.0g/dL peripheral blood, or higher level. In some embodiments, the fetal hemoglobin produced by the modified CD34-positive HSPCs after differentiation is about 9.0-18 g/dL, e.g., 10-17 g/dL, 11-15 g/dL, or 12-16 g/dL peripheral blood.

In some embodiments of the above method, as for the desired level of γ-globin, the modified CD34-positive HSPCs produces more than about 50%, 60%, 70%, 80%, 90%, 100%, 120%, 150%, 200%, 250%, 300%, 350%, 400%, 450%, 500%, 550%, 600%, or higher level of γ-globulin mRNA as compared with the unmodified CD34-positive HSPCs.

In some embodiments of the above method, the evaluation step comprises:
a) isolating CD34-positive HSPCs from the bone marrow or peripheral blood sample of the individual to obtain an isolated CD34-positive HSPCs population ("isolated EV cells");
b) modifying the isolated EV cells to obtain a first population of the modified CD34-positive HSPCs cells with reduced BCL11A function ("modified EV cells"); and
c) evaluating the ability of the modified EV cells to produce a desired level of γ-globin or fetal hemoglobin (HbF) after differentiation.

In some embodiments of the above method, the treatment step comprises:
a) mobilize the CD34-positive HSPCs in the bone marrow of the individual into the peripheral blood to increase the amount of CD34-positive HSPCs in the peripheral blood;
b) isolating CD34-positive HSPCs from the peripheral blood of the individual to obtain an isolated CD34-positive HSPCs population ("isolated TR cells"),
c) modifying the isolated TR cells to obtain a second population of the modified CD34-positive HSPCs with reduced BCL11A function ("modified TR cells"); and
d) administering an effective amount of the modified TR cells to the individual.

In one aspect, the present application provides a method for treating hemoglobinopathy in an individual, which comprises:
1) an evaluation step, comprising:
   a) isolating CD34-positive HSPCs from the bone marrow or peripheral blood sample of the individual to obtain an isolated CD34-positive HSPCs population ("isolated EV cells");
   b) modifying the isolated EV cells to obtain a first population of the modified CD34-positive HSPCs cells with reduced BCL11A function ("modified EV cells"); and
   c) evaluating the ability of the modified EV cells to produce a desired level of γ-globin or fetal hemoglobin (HbF) after differentiation; and
2) a treatment step, comprising:
   a) mobilizing the CD34-positive HSPCs in the individual to the peripheral blood;
   b) isolating CD34-positive HSPCs from the peripheral blood of the individual to obtain an isolated CD34-positive HSPCs population ("isolated TR cells"),
   c) modifying the isolated TR cells to obtain a second population of the modified CD34-positive HSPCs with reduced BCL11A function ("modified TR cells"); and
   d) administering an effective amount of the modified TR cells to the individual.

In one aspect, the present application provides a method for treating hemoglobinopathy in an individual, which comprises:
1) an evaluation step, comprising:
   a) isolating CD34-positive HSPCs from the bone marrow or peripheral blood sample of the individual to obtain an isolated CD34-positive HSPCs population ("isolated EV cells");
   b) genetically modifying (e.g., genetic modification by the CRISPR method) the isolated EV cells to obtain a first population of the modified CD34-positive HSPCs cells with reduced BCL11A function ("modified EV cells"); and
   c) evaluating the ability of the modified EV cells to produce a desired level of γ-globin or fetal hemoglobin (HbF) after differentiation; and
2) a treatment step, comprising:
   a) mobilizing the CD34-positive HSPCs in the individual to the peripheral blood;
   b) isolating CD34-positive HSPCs from the peripheral blood of the individual to obtain an isolated CD34-positive HSPCs population ("isolated TR cells"),
   c) genetically modifying (e.g., genetic modification by the CRISPR method) the isolated TR cells to obtain a second population of the modified CD34-positive HSPCs with reduced BCL11A function ("modified TR cells"); and
   d) administering (e.g., by intravenous injection, including a single intravenous injection) an effective amount of the modified TR cells to the individual.

In some embodiments of the above method, the bone marrow or peripheral blood sample used for isolating CD34-positive HSPCs to produce isolated EV cells is a small amount, for example, the sampling volume of the bone marrow is not more than 20 ml, e.g., 5-20 ml, 5-10 ml; the sampling volume of the peripheral blood is not more than 30ml, e.g., 10-30ml, 15-20ml.

In some embodiments of the above method, the bone marrow or peripheral blood sample used for isolating CD34-positive HSPCs to produce isolated TR cells is a large amount, for example, not less than 50 mL, e.g., 50-300 ml, 100-200 ml.

In some embodiments of the above method, the CD34-positive HSPCs cells may be obtained from the bone marrow or peripheral blood of the individual. In some embodiments, the separation method includes magnetic bead separation. In some embodiments of the above method, the method comprises genetically modifying the isolated EV cells. In some embodiments of the above method, the genetic modification includes genetically modifying the isolated EV cells by any method selected from the group consisting of: zinc finger nuclease (ZFN), transcription activator-like effector nuclease (TALEN), and clustered regularly interspaced short palindromic repeats (CRISPRs), RNA editing, RNA interference technology, or a combination thereof. In some embodiments of the above method, BCL11A function is reduced by modifying the BCL11A gene in the region of positions 60495197-60495346 on human chromosome 2. In some embodiments of the above method, BCL11A function is reduced through genetically modifying the CD34-positive HSPCs cells by CRISPR/Cas technology. In some embodiments of the above method, BCL11A function is reduced through modifying the CD34-positive HSPCs cells by BCL11A function inhibitor. In some embodiments, the BCL11A function inhibitor is a protein or nucleic acid molecule inhibiting the transcription and/or expression of the BCL11A gene, e.g., nuclease (such as ZFN and TALEN), peptide nucleic acid, antisense RNA, siRNA, miRNA, and shRNA. In some embodiments, the BCL11A function inhibitor is a protein or nucleic acid molecule inhibiting the transcription and/or expression of a gene in the region of positions 60495197-60495346 on human chromosome 2. In some embodiments, the BCL11A function inhibitor is a protein or nucleic acid molecule interfering, inhibiting, or destroying the transcription and/or expression of a gene in the region of positions 60495197-60495346 on human chromosome 2. In some embodiments, the BCL11A function inhibitor is nuclease (such as ZFN and TALEN), peptide nucleic acid, antisense RNA, siRNA, miRNA interfering, inhibiting, or destroying the transcription and/or expression of a gene in the region of positions 60495197-60495346 on human chromosome 2.

In some embodiments of the above method, the ability of the modified EV cells to produce a desired level of γ-globin or fetal hemoglobin (HbF) after differentiation is evaluated in the evaluation step comprising: 1) culturing the modified EV cells under conditions allowing differentiation to obtain an erythrocyte population; and 2) determining the level of γ-globulin or fetal hemoglobin (HbF) produced by the erythrocytes.

In some embodiments of the above method, the ability of the modified EV cells to produce a desired level of γ-globin or fetal hemoglobin (HbF) after differentiation is evaluated in the evaluation step comprising: determining the mRNA level of γ-globulin. In some embodiments of the above method, the ability of the modified EV cells to produce a desired level of γ-globin or fetal hemoglobin (HbF) after differentiation is evaluated in the evaluation step comprising: determining the protein level of fetal hemoglobin (HbF). In some embodiments of the above method, wherein the individual has not undergone CD34-positive HSPCs cell mobilization or pretreatment prior to the evaluation step, for example, the individual has not been injected an agent for clearing bone marrow and lymph (such as Busulfan and Fludarabine) prior to the evaluation step. In some embodiments of the above method, the evaluation step is repeated at least once prior to the treatment step.

The method for modifying the isolated TR cells may be the same as or different from the method for modifying the isolated EV cells. In some embodiments, the method for modifying the isolated TR cells may be the same as the method for modifying the isolated EV cells. In some embodiments of the above method, the isolated TR cells are genetically modified. In some embodiments of the above method, the genetic modification comprises genetically modifying the isolated TR cells by any method selected from the group consisting of: zinc finger nuclease (ZFN), transcription activator-like effector nuclease (TALEN), and clustered regularly interspaced short palindromic repeats (CRISPRs), RNA editing, RNA interference (RNAi), or a combination thereof. In some embodiments of the above method, BCL11A function is reduced by modifying the BCL11A gene in the region of positions 60495197-60495346 on human chromosome 2. In some embodiments of the above method, BCL11A function is reduced through genetically modifying the CD34-positive HSPCs cells by CRISPR/Cas technology. In some embodiments of the above method, BCL11A function is reduced through modifying the CD34-positive HSPCs cells by BCL11A function inhibitor. In some embodiments, the BCL11A function inhibitor is a protein or nucleic acid molecule inhibiting the transcription and/or expression of the BCL11A gene, e.g., nuclease (such as ZFN and TALEN), peptide nucleic acid, antisense RNA, siRNA, miRNA, and shRNA. In some embodiments, the BCL11A function inhibitor is a protein or nucleic acid molecule inhibiting the transcription and/or expression of a gene in the region of positions 60495197-60495346 on human chromosome 2. In some embodiments, the BCL11A function inhibitor is a protein or nucleic acid molecule interfering, inhibiting, or destroying the transcription and/or expression of a gene in the region of positions 60495197-60495346 on human chromosome 2. In some embodiments, the BCL11A function inhibitor is nuclease (such as ZFN and TALEN), peptide nucleic acid, antisense RNA, siRNA, miRNA interfering, inhibiting, or destroying the transcription and/or expression of a gene in the region of positions 60495197-60495346 on human chromosome 2.

In some embodiments, the treatment step comprises mobilizing the bone marrow of the individual so that a large amount of hematopoietic stem cells is produced by the bone marrow and released into the peripheral blood circulatory system. Mobilizing CD34-positive HSPCs comprises administering granulocyte colony stimulating factor (GCSF) and/or plerixafor to the individual (for example, 4-10 days, 5-8 days, or 6-7 days) prior to collecting CD34-positive HSPCs cells.

In some embodiments of the above method, the treatment step further comprises: pretreating the individual prior to administering the modified TR cells. Pretreatment may include a therapy of clearing bone marrow and/or clearing lymph. In some embodiments, the pretreatment comprises chemotherapy, monoclonal antibody therapy, or systemic radiation. In some embodiments, the chemotherapy comprises administering to the individual one or more chemotherapeutic agents selected from the group consisting of: Busulfan, Cyclophosphamide and Fludarabine.

In some embodiments of the above method, the treatment step comprises administering (e.g., by intravenous injection, comprising a single intravenous injection) to the individual ≥2×10⁶, ≥5×10⁶, ≥1×10⁷, or ≥2×10⁷ cells/kg body weight of the modified TR cells.

In some embodiments, the isolated TR cells are cultured for one or more days prior to modification, and then the isolated TR cells are modified. In some embodiments, the modified TR cells are cultured for one or more days (e.g., 2 days, 3 days, 4 days, 5 days, 6 days, 7 days, or 8 days) prior to being administered to the individual. In some embodiments, the modified TR cells are stored under a freezing condition for at least 24 hours prior to administering the modified TR cells to the individual. In some embodiments, the modified TR cells are cultured for one or more days (e.g., 2 days, 3 days, 4 days, 5 days, 6 days, 7 days, or 8 days) prior to being stored under a freezing condition.

In some embodiments of the above method, the hemoglobinopathy is selected from the group consisting of: sickle cell disease, sickle cell trait, hemoglobin C disease, hemoglobin C trait, hemoglobin S/C disease, hemoglobin D disease, hemoglobin E disease, thalassemia, hemoglobin-related disorder with increased oxygen affinity, hemoglobin-related disorder with decreased oxygen affinity, unstable hemoglobin disease, and methemoglobinemia. In some embodiments, the hemoglobinopathy is selected from the group consisting of: β-thalassemia and sickle cell anemia. In some embodiments, the hemoglobinopathy is β⁰ or β⁺ thalassemia.

In some embodiments of the above method, the individual is a human. In some embodiments, the individual is a human under 35 years of age. In some embodiments, the individual is a male. In some embodiments, the individual is a female. In some embodiments, the individual is a human without complications of the heart, liver, lung, or spleen. In some embodiments, the hemoglobin level of the individual before treatment is no more than 5.0 g/dL, 6.0 g/dL, 7.0 g/dL, 8.0 g/dL, or 9.0 g/dL peripheral blood. In some embodiments of the above method, the treatment step is performed immediately following the evaluation step.

### BRIEF DESCRIPTION OF THE DRAWINGS

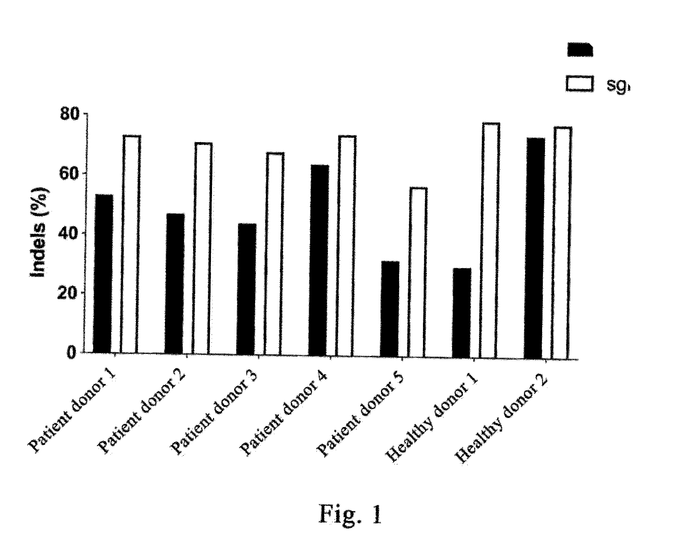

**Fig. 1****:** 2 sgRNAs of Cas9 mRNA coding gene (SEQ ID NO: 1) and destroyed BCL11A erythroid enhancer (respectively sgRNA-1 and sgRNA-2) are introduced into the hematopoietic stem cells respectively derived from mobilized peripheral blood of 5 different thalassemia patients and 2 healthy donors by electroporation, 4 days later, the statistical analysis on the efficiency of generating Indels is performed by "Synthego ICE Analysis" online software.

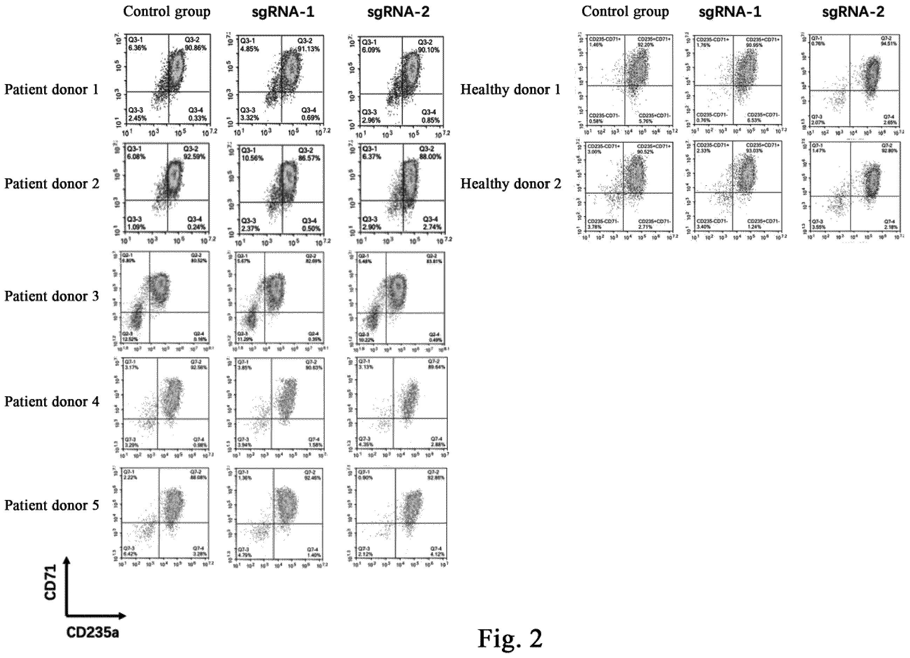

**Fig. 2:** 2 sgRNAs of Cas9 mRNA and destroyed BCL11A erythroid enhancer (respectively sgRNA-1 and sgRNA-2) are introduced into the CD34-positive hematopoietic stem cells respectively derived from mobilized peripheral blood of 5 different thalassemia patients and 2 healthy donors by electroporation, performing erythrocyte differentiation, detecting 18 days later to find the expression ratio of the two membrane proteins of human CD71 and human CD235a, which represents the efficiency of erythroid differentiation. Control group: representing cells without undergoing gene editing. sgRNA-1 and sgRNA-2: representing two kinds of cells respectively having two kinds of sgRNA undergoing gene editing.

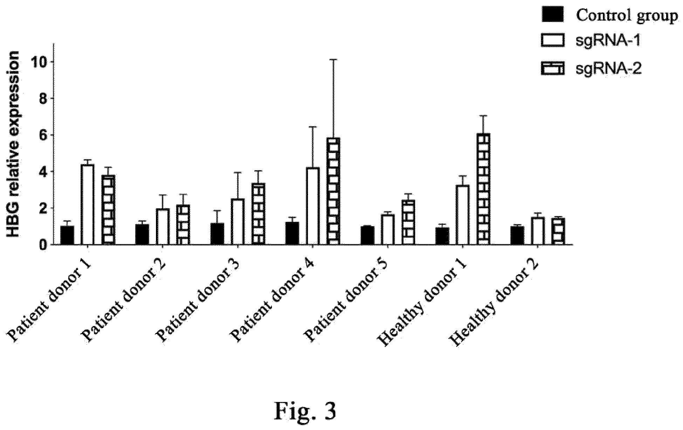

**Fig. 3****:** 2 sgRNAs of Cas9 mRNA and destroyed BCL11A erythroid enhancer (respectively sgRNA-1 and sgRNA-2) are introduced into the CD34-positive hematopoietic stem cells respectively derived from mobilized peripheral blood of 5 different thalassemia patients and 2 healthy donors by electroporation, performing erythrocyte differentiation, 18 days later detecting the expression of HBG (y-globin) gene mRNA by fluorescence quantitative PCR. Control group: representing cells without undergoing gene editing. Experimental group: representing two kinds of cells respectively undergoing gene editing of two kinds of sgRNA. N=3 experimental replicates. HBG gene and GAPDH gene are normalized.

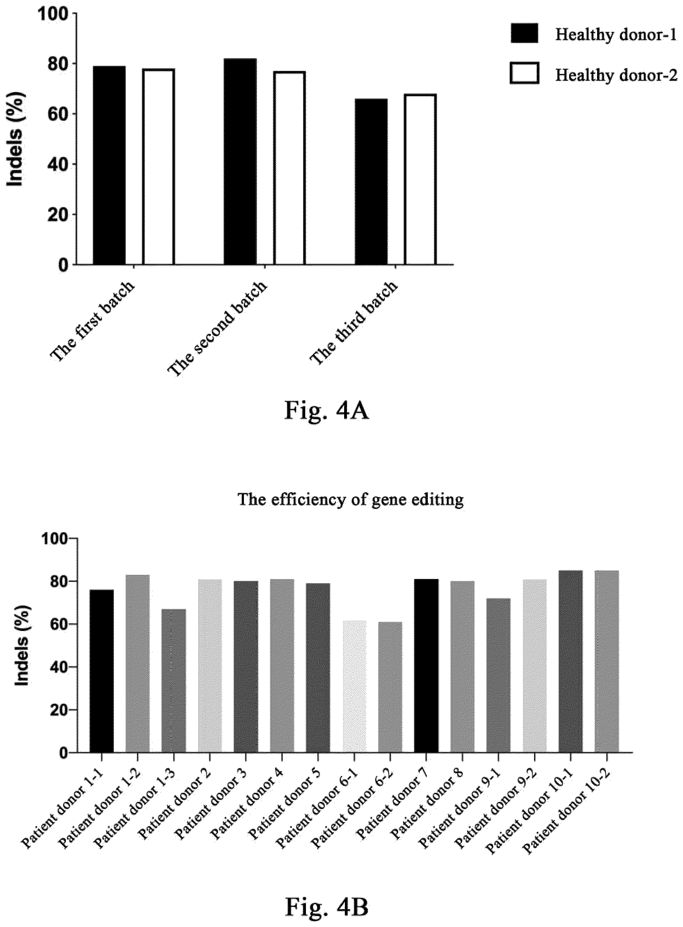

**Fig. 4A****:** Cas9 mRNA and sgRNA-2 of destroyed BCL11A erythroid enhancer are introduced into the CD34-positive hematopoietic stem cells respectively derived from mobilized peripheral blood of 2 different healthy donors by electroporation, performing 3 batches of experiments, 4 days later, the statistical analysis on the efficiency of generating Indels is performed by "Synthego ICE Analysis" online software. **Fig. 4B****:** Cas9 mRNA and sgRNA-2 of destroyed BCL11A erythroid enhancer are introduced into the CD34-positive hematopoietic stem cells respectively derived from mobilized peripheral blood of 10 thalassemia patients by electroporation, performing multiple batches of experiments (patient donor 1, three batches; patient donor 6, patient donor 9, and patient donor 10, respectively two independent batches), 2 days later, the statistical analysis on the efficiency of generating Indels is performed by "Synthego ICE Analysis" online software.

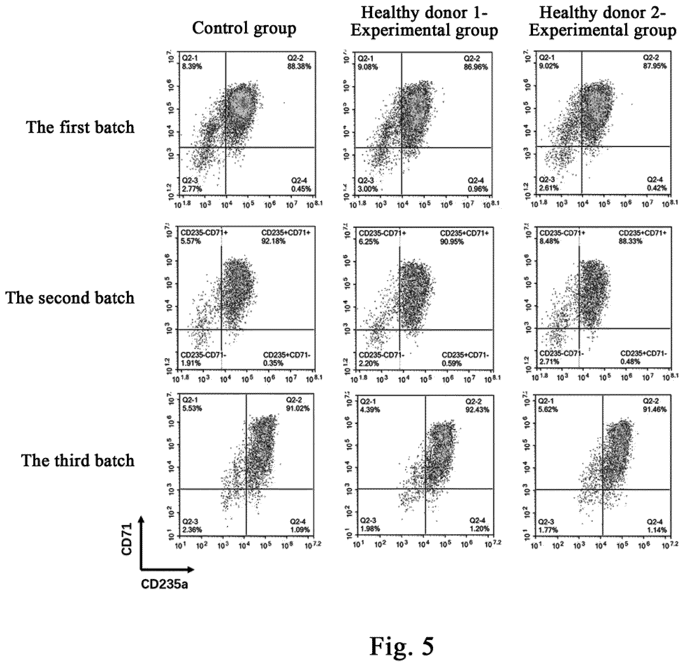

**Fig. 5****:** Cas9 mRNA and sgRNA-2 of destroyed BCL11A erythroid enhancer are introduced into the CD34-positive hematopoietic stem cells respectively derived from mobilized peripheral blood of 2 different healthy donors by electroporation, performing 3 batches of experiments; erythrocyte differentiation is conducted, 18 days later detecting the expression of the two membrane proteins of human CD71 and human CD235a to indicate the efficiency of erythroid differentiation. Control group: representing cells without undergoing gene editing. Experimental group: representing cells undergoing gene editing of sgRNA-2.

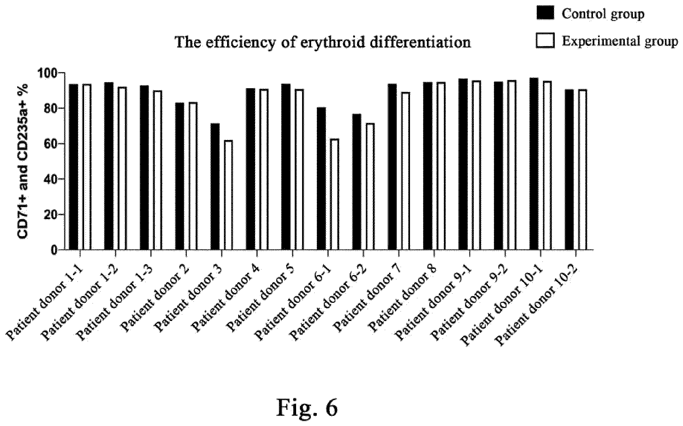

**Fig. 6****:** Cas9 mRNA and sgRNA-2 of destroyed BCL11A erythroid enhancer are introduced into the CD34-positive hematopoietic stem cells respectively derived from mobilized peripheral blood of 10 thalassemia patients by electroporation, performing multiple batches of experiments; erythrocyte differentiation is conducted, 18 days later detecting the expression of the two membrane proteins of human CD71 and human CD235a to indicate the efficiency of erythroid differentiation. Control group: representing cells without undergoing gene editing. Experimental group: representing cells undergoing gene editing of sgRNA-2.

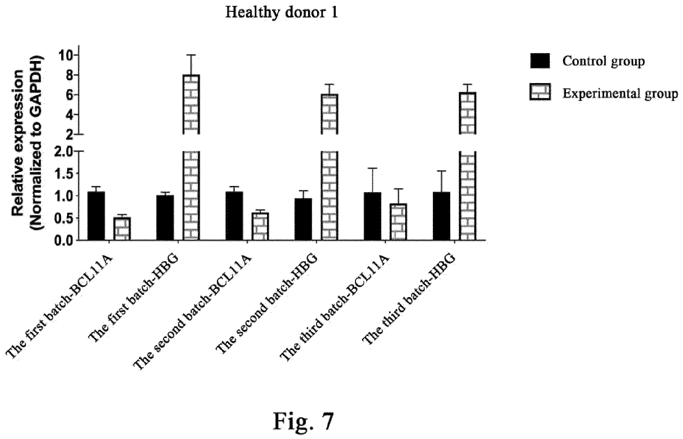

**Fig. 7****:** Cas9 mRNA and sgRNA-2 of destroyed BCL11A erythroid enhancer are introduced into the CD34-positive hematopoietic stem cells respectively derived from mobilized peripheral blood of the first healthy donor by electroporation, performing 3 batches of erythrocyte differentiation experiments; 18 days after differentiation, detecting the mRNA expression of the genes comprising BCL11A and HBG (y-globin) by fluorescence quantitative PCR. Control group: representing cells without undergoing gene editing. Experimental group: representing cells undergoing gene editing of sgRNA-2. N=3 experimental replicates. HBG gene and BCL11A gene are respectively normalized with GAPDH gene.

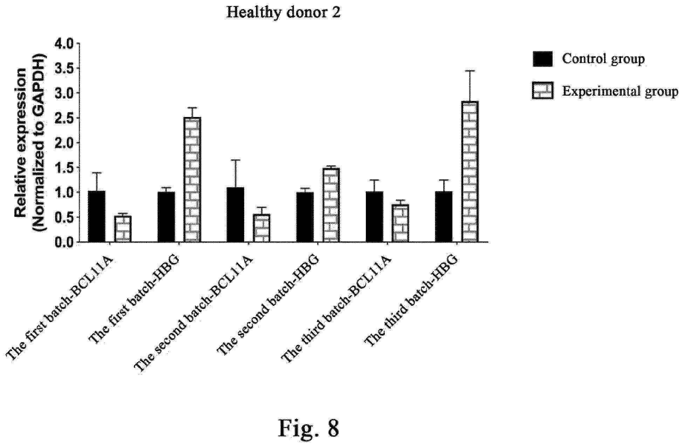

**Fig. 8****:** Cas9 mRNA and sgRNA-2 of destroyed BCL11A erythroid enhancer are introduced into the CD34-positive hematopoietic stem cells respectively derived from mobilized peripheral blood of the second healthy donor by electroporation, performing 3 batches of erythrocyte differentiation experiments; 18 days after differentiation, detecting the mRNA expression of the genes comprising BCL11A and HBG (y-globin) by fluorescence quantitative PCR. Control group: representing cells without undergoing gene editing. Experimental group: representing cells undergoing gene editing of sgRNA-2. N=3 experimental replicates. HBG gene and BCL11A gene are respectively normalized with GAPDH gene.

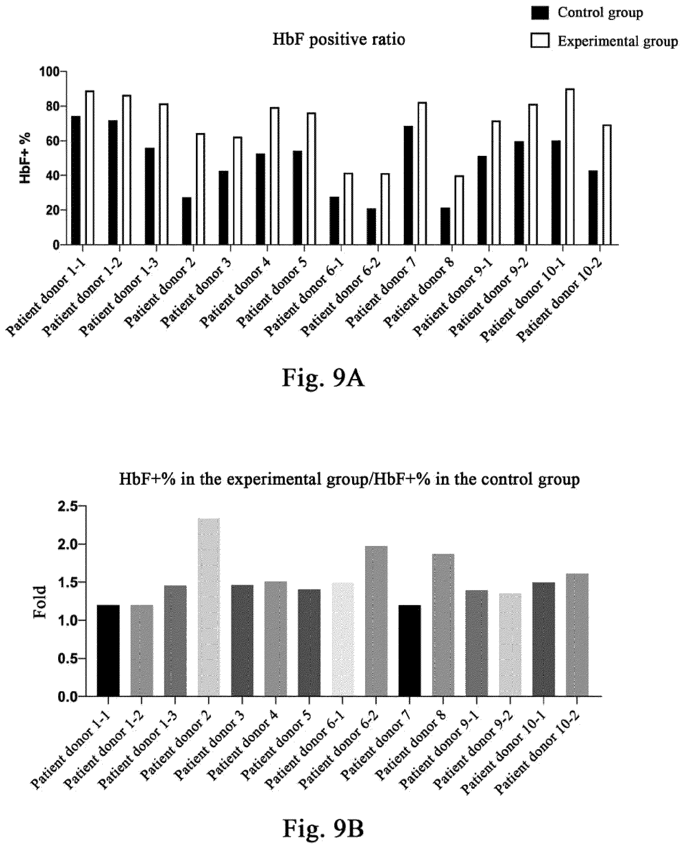

**Fig. 9A****:** Cas9 mRNA and sgRNA-2 of destroyed BCL11A erythroid enhancer are introduced into the CD34-positive hematopoietic stem cells respectively derived from mobilized peripheral blood of 10 thalassemia patients by electroporation, performing multiple batches of experiments; erythrocyte differentiation is conducted, 18 days later detecting the ratio of HbF expression in erythrocytes after differentiation by flow cytometry. Control group: representing cells without undergoing gene editing. Experimental group: representing cells undergoing gene editing of sgRNA-2. **Fig. 9B****:** Statistical analysis of the multiples of HbF+% in the experimental group and HbF+% in the control group from different thalassemia patients.

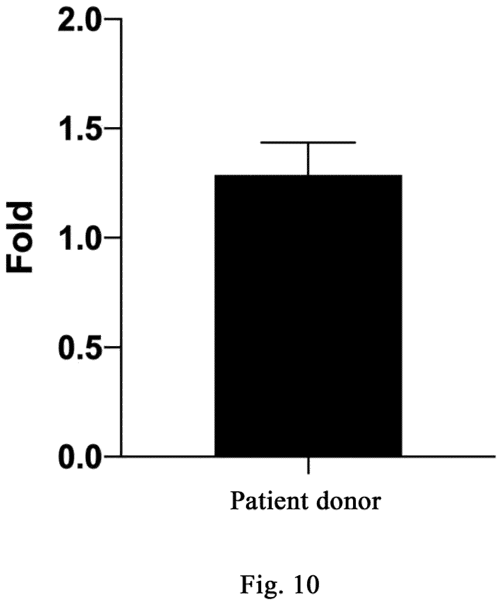

**Fig. 10****:** Statistical analysis of the multiples of HbF+% in the experimental group and HbF+% in the control group from thalassemia patient 1, n=3 experimental replicates.

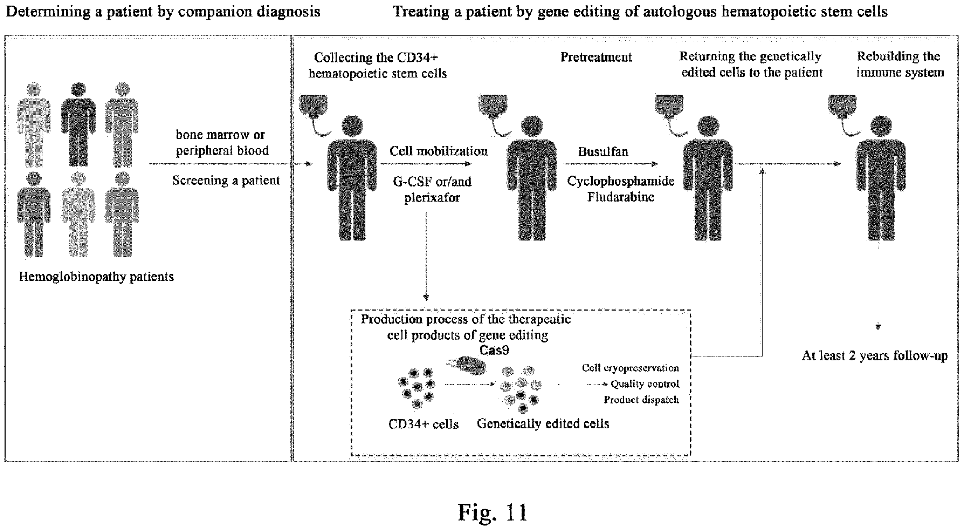

**Fig. 11****:** Schematic diagram of a novel therapeutic solution of gene editing BCL11A erythroid enhancer site for treating hemoglobinopathy.

### SPECIFIC EMBODIMENTS

This application relates to a method for predicting whether gene editing technology is effective or the effectiveness level of gene editing in treating hemoglobinopathy (e.g., β-thalassemia and sickle cell anemia) by reducing BCL11A function, for example, by destroying BCL11A erythroid enhancer site in hematopoietic stem cells through genome editing technology, conducting erythrocyte differentiation on the hematopoietic stem cells undergoing gene editing, and evaluating the degree of up-regulation of the expression of γ-globin and fetal hemoglobin.

Particularly, the present application provides a method for treating hemoglobinopathy in an individual, which comprises:
a) an evaluation step comprising: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells"); and
b) a treatment step comprising: administering a second population of the modified CD34-positive HSPCs to the individual, wherein the modified CD34-positive HSPCs are derived from the individual and are modified to reduce BCL11A function ("modified TR cells"). In the embodiments of the above method, the first population and/or the second population are modified to reduce BCL11A function by modifying the BCL11A gene in the region of positions 60495197-60495346 on human chromosome 2 (e.g., genetic modification by CRISPR/Cas technology, comprising introducing sgRNA comprising a sequence selected from any one of SEQ ID NOs: 3-25 into the CD34-positive hematopoietic stem cells/progenitor cells to edit the BCL11A gene).

In one aspect, the present application provides a method for treating hemoglobinopathy in an individual, which comprises a treatment step comprising: administering a second population of the modified CD34-positive HSPCs to the individual, wherein the modified CD34-positive HSPCs are derived from the individual and are modified to reduce BCL11A function ("modified TR cells"), and
wherein the individual is selected for treatment based on the functional evaluation from an evaluation step which comprises: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells"). In the embodiments of the above method, the first population and/or the second population are modified to reduce BCL11A function by modifying the BCL11A gene in the region of positions 60495197-60495346 on human chromosome 2 (e.g., genetic modification by CRISPR/Cas technology, comprising introducing sgRNA comprising a sequence selected from any one of SEQ ID NOs: 3-25 into the CD34-positive hematopoietic stem cells/progenitor cells to edit the BCL11A gene).

In one aspect, the present application provides a method for selecting an individual suffering from hemoglobinopathy for treatment with a second population of the modified CD34-positive HSPCs which are derived from the individual and are modified to reduce BCL11A function ("modified TR cells"), wherein the method comprises an evaluation step comprising: evaluating the ability of a first population of the modified CD34-positive HSPCs to produce a desired level of γ -globin or fetal hemoglobin (HbF) after differentiation, wherein the modified CD34-positive HSPCs of the first population are derived from the individual and are modified to reduce BCL11A function ("modified EV cells"), wherein if the first population of the modified CD34-positive HSPCs produce a desired level of γ-globin or fetal hemoglobin (HbF), the individual is selected for treatment. In the embodiments of the above method, the first population and/or the second population are modified to reduce BCL11A function by modifying the BCL11A gene in the region of positions 60495197-60495346 on human chromosome 2 (e.g., genetic modification by CRISPR/Cas technology, comprising introducing sgRNA comprising a sequence selected from any one of SEQ ID NOs: 3-25 into the CD34-positive hematopoietic stem cells/progenitor cells to edit the BCL11A gene).

In one aspect, the present application provides a method for treating hemoglobinopathy in an individual, which comprises:
1) an evaluation step, comprising:
   a) isolating CD34-positive HSPCs from the bone marrow or peripheral blood sample of the individual to obtain an isolated CD34-positive HSPCs population ("isolated EV cells");
   b) genetically modifying (e.g., genetic modification by the CRISPR method) the isolated EV cells to obtain a first population of the modified CD34-positive HSPCs cells with reduced BCL11A function ("modified EV cells"); and
   c) evaluating the ability of the modified EV cells to produce a desired level of γ-globin or fetal hemoglobin (HbF) after differentiation; and
2) a treatment step, comprising:
   a) mobilizing the CD34-positive HSPCs in the individual to the peripheral blood;
   b) isolating CD34-positive HSPCs from the peripheral blood of the individual to obtain an isolated CD34-positive HSPCs population ("isolated TR cells"),
   c) genetically modifying (e.g., genetic modification by the CRISPR method) the isolated TR cells to obtain a second population of the modified CD34-positive HSPCs with reduced BCL11A function ("modified TR cells"); and
   d) administering (e.g., by intravenous injection, including a single intravenous injection) an effective amount of the modified TR cells to the individual. In the embodiments of the above method, the first population and/or the second population are modified to reduce BCL11A function by modifying the BCL11A gene in the region of positions 60495197-60495346 on human chromosome 2 (e.g., genetic modification by CRISPR/Cas technology, comprising introducing sgRNA comprising a sequence selected from any one of SEQ ID NOs: 3-25 into the CD34-positive hematopoietic stem cells/progenitor cells to edit the BCL11A gene).

In some embodiments of the above method, the bone marrow or peripheral blood sample used for isolating CD34-positive HSPCs to produce isolated EV cells is a small amount, for example, the sampling volume of the bone marrow is not more than 20 ml, e.g., 5-20 ml, 5-10 ml; the sampling volume of the peripheral blood is not more than 30ml, e.g., 10-30ml, 15-20ml.

In some embodiments of the above method, the bone marrow or peripheral blood sample used for isolating CD34-positive HSPCs to produce isolated TR cells is a large amount, for example, not less than 50 mL, e.g., 50-300 ml, 100-200 ml.

In some embodiments of the above method, the CD34-positive HSPCs cells may be isolated from the bone marrow or peripheral blood of the individual. In some embodiments, the separation method comprises magnetic bead separation. For example, cells in bone marrow or peripheral blood are specifically labeled with super paramagnetic MACS MicroBeads. After magnetic labeling, these cells are passed through a sorting column placed in a strong and stable magnetic field (the matrix in the sorting column creates a high gradient magnetic field). The magnetically labeled cells stay in the column while the unlabeled cells flow out. When the sorting column is removed from the magnetic field, the magnetically labeled cells in the column may be eluted, so that two cell populations of labeled and unlabeled cells may be obtained. In a particular embodiment, the Ficoll liquid density gradient centrifugation may be used to separate different cell layers in the blood by low-speed density gradient centrifugation utilizing the difference in the specific gravity of different components. The density of erythrocytes and granulocytes is greater than that of the stratified liquid, and the erythrocytes will quickly agglomerate into a string and accumulate at the bottom of the tube after encountering the Ficoll liquid. Only the mononuclear cells with the same density as the stratification liquid are enriched between the plasma layer and the stratified liquid, i.e., the buffy coat, and the hematopoietic stem cells exist in this layer. CD34-positive HSPCs cells may be obtained by subsequent magnetic bead sorting.

In one aspect, the application provides a method for determining whether an individual suffering from hemoglobinopathy is suitable for treating with a second population of the modified CD34-positive HSPCs ("modified TR cells") derived from the individual and modified to reduce BCL11A function, wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), and wherein if the first population of the modified CD34-positive HSPCs produce a desired level of γ-globin or fetal hemoglobin (HbF), the individual is suitable for treatment.

In one aspect, the application provides a method for determining whether an individual suffering from hemoglobinopathy is unsuitable for treating with a second population of the modified CD34-positive HSPCs ("modified TR cells") derived from the individual and modified to reduce BCL11A function, wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), wherein if the first population of the modified CD34-positive HSPCs do not produce a desired level of γ-globin or fetal hemoglobin (HbF), the individual is not suitable for treating with the second population of the modified CD34-positive HSPCs ("modified TR cells").

"Hematopoietic stem cells" refer to a cell population with vigorous proliferation potential, multi-differentiation ability and self-renewal ability. Hematopoietic stem cells may not only differentiate into and supplement various blood cells, but also maintain the characteristics and quantity of stem cells through self-renewal. The differentiation degree and proliferation ability of hematopoietic stem cells are different and heterogeneous. Pluripotent hematopoietic stem cells are the most primitive, firstly they differentiate into directed pluripotent hematopoietic stem cells, e.g., myeloid hematopoietic stem cells capable of generating granulocytes, erythrocytes, mononuclear cells and megakaryocyte-platelet cells, and lymphocyte stem cells capable of generating B lymphocytes and T lymphocytes. These two types of stem cells maintain the basic characteristics of hematopoietic stem cells, and are also slightly differentiated; and they are respectively responsible for "bone marrow components" and the occurrence of lymphocytes, thus they are called directed pluripotent hematopoietic stem cells. They further differentiate into hematopoietic progenitor cells which are also primitive blood cells but have lost many of the basic characteristics of hematopoietic stem cells, for example, losing the multi-differentiation ability and only differentiating towards one cell line or closely related two cell lines; losing the ability to renew itself repeatedly, and relying on the proliferation and differentiation of hematopoietic stem cells to supplement the number; having limited proliferation potential, and only dividing a few times. According to the number of blood cell lines differentiated from hematopoietic progenitor cells, they are divided into unipotent hematopoietic progenitor cells (differentiating into only one blood cell line) and oligopotent hematopoietic progenitor cells (differentiating into 2 to 3 blood cell lines). In this application, the terms "hematopoietic stem cell/progenitor cell" and "hematopoietic stem cell" may be used interchangeably, covering pluripotent hematopoietic stem cells, directed pluripotent hematopoietic stem cells and hematopoietic progenitor cells, and they are the general terms for hematopoietic stem cells with different heterogeneities.

The "CD34-positive hematopoietic stem cells/progenitor cells" mentioned in this application, abbreviated as CD34-positive HSPCs, refers to a population of stem cells and progenitor cells that express CD34 markers on the surface and have hematopoietic function. For example, CD34-positive hematopoietic stem/progenitor cells may be detected and counted, for example, by flow cytometry and fluorescently labeled anti-CD34 antibodies.

In a particular embodiment, CD34-positive hematopoietic stem cells/progenitor cells are isolated or obtained from an organism (individual) comprising cells of hematopoietic origin. "Separation" refers to removal from its original environment. For example, a cell is isolated if it is separated from some or all of the components normally accompany it in its natural state.

Hematopoietic stem cells/progenitor cells may be obtained or isolated from unfractionated or fractionated bone marrow of adults, the sources includes femurs, hip bones, ribs, sternum and other bones. Hematopoietic stem cells and progenitor cells may be directly obtained or separated from hip bones with a needle and syringe, or obtained from the blood, usually obtained from the blood after pretreatment with a hematopoietic stem cell mobilizer such as GCSF (granulocyte colony stimulating factor). Other sources of hematopoietic stem cells and progenitor cells include cord blood, placental blood, and mobilized peripheral blood of individuals.

After a cell population is isolated and obtained from an individual (such as bone marrow or peripheral blood), it may be further purified to obtain CD34-positive hematopoietic stem cells/progenitor cells, for example, removing mature lineage-directed cells in an isolated cell population by immunization, e.g., labelling the solid matrix by antibodies binding to a set of "lineage" antigens (e.g., CD2, CD3, CD11b, CD14, CD15, CD16, CD19, CD56, CD123, and CD235a), and then separating the original hematopoietic stem cells and progenitor cells with antibodies binding to the CD34-positive antigens. Kits for purifying hematopoietic stem cells and progenitor cells from a variety of cell sources are commercially available, and in particular embodiments, these kits may be used together with the methods of the present invention.

"CD34 positive hematopoietic stem cells/progenitor cells" may represent at least 50%, 60%, 70%, 80%, 90%, 95%, 96%, 97%, 98%, 99%, or 100% of the CD34-positive hematopoietic stem cell/progenitor cell population in a cell population rich in CD34 positive cells.

A "modified" cell refers to a cell undergoing changes at the molecular or cellular level through biological or chemical methods. For example, the isolated CD34-positive hematopoietic stem cells/progenitor cells ("CD34-positive HSPCs") destroy the BCL11A gene or its coding RNA to produce the modified EV cells or modified TR cells with reduced BCL11A function by gene editing methods, for example, by any method selected from the group consisting of: ZFN, TALEN, CHRISPR, RNA editing, or RNAi technology. BCL11A function inhibitors including nucleases (such as ZFN and TALEN), peptide nucleic acid, antisense RNA, siRNA, miRNA, and shRNA may also be used to inhibit the function of BCL11A gene so as to modify the isolated CD34-positive hematopoietic stem cells/progenitor cells (" CD34-positive HSPCs"), thereby producing modified EV cells or modified TR cells. CD34 positive

"EV cell" as used herein represents a cell used for evaluation (EV); and "TR cell" as used herein represents a cell used for treatment (TR).

The "mobilization" of hematopoietic stem cells refers to the process by which the hematopoietic stem cells in the hematopoietic microenvironment move from a specific bone marrow microenvironment to the peripheral circulation after being affected by a mobilizing agent.

Hemoglobinopathy is a group of inherited blood diseases caused by abnormal hemoglobin molecular structure or abnormal synthesis of globin peptide chain. Clinical manifestations include hemolytic anemia, methemoglobinemia, or tissue hypoxia caused by increased or decreased oxygen affinity of hemoglobin, or cyanosis due to compensatory erythrocytosis.

"Hemoglobin" is a binding protein consisting of globin and heme. The globin molecule has two pairs of peptide chains, one pair are α chains, the other pair are non-a chains, comprising 5 kinds of chains, i.e., β, γ, δ, and ξ chain (with a structure similar to α chain), and ε chain. Human hemoglobin is a tetramer consisting of two pairs (4) of hemoglobin monomers (each peptide chain is connected to a heme to form a hemoglobin monomer) combined according to a certain spatial relationship, e.g., HbA (or HbA1, α2β2), HbA2 (α2δ2), and HbF (α2γ2), wherein HbF (α2γ2) is a tetramer of fetal hemoglobin.

As used herein, "BCL11A" is a transcription factor. It was first found in mice as a binding site for retroviruses and was named Evi9. Later, this gene was also found in the human genome and located on 2p13 site of the short arm of chromosome 2.

Reduced BCL11A function after modification means that BCL11A gene is destroyed or its expression and/or transcription is inhibited, attenuated, blocked, and interfered by modification, e.g., genetic modification (for example, gene editing at the DNA level and/or RNA level) or by adding BCL11A function inhibitors (for example, nucleases (such as ZFN and TALEN), peptide nucleic acid, antisense RNA, siRNA, miRNA, or shRNA targeting the BCL11A gene).

As used herein, "CRISPR/Cas" is a gene editing technology, including but not limited to various naturally occurring or artificially designed CRISPR/Cas systems, such as the CRISPR/Cas9 system. The naturally occurring CRISPR/Cas system is an adaptive immune defense formed during the long-term evolution of bacteria and archaea, which may be used to fight the invading viruses and foreign DNA. For example, the working principle of CRISPR/Cas9 is that crRNA (CRISPR-derived RNA) combines with tracrRNA (trans-activating RNA) through base pairing to form a tracrRNA/crRNA complex, which guides the nuclease Cas9 protein to cut the double-stranded DNA at the target site of a sequence pairing with crRNA. sgRNA for guidance (single guide RNA) may be formed by artificially designing tracrRNA and crRNA, and it is sufficient to guide Cas9 to perform a site-specific cleavage of DNA. As an RNA-guided dsDNA binding protein, Cas9 effector nuclease may co-localize RNA, DNA and protein, and it has great potential for transformation. The CRISPR/Cas system may use type 1, type 2 or type 3 Cas proteins. In some embodiments of the present invention, the method uses Cas9. Other applicable CRISPR/Cas systems include but are not limited to the systems and methods described in WO2013176772, WO2014065596, WO2014018423, US8,697,359, PCT/CN2018/112068, and PCT/CN2018/112027.

In some embodiments of the above method, the method comprises genetically modifying the isolated EV cells. In some embodiments of the above method, the genetic modification comprises:
genetically modifying the isolated EV cells by any method selected from the group consisting of: zinc finger nuclease (ZFN), transcription activator-like effector nuclease (TALEN), and clustered regularly interspaced short palindromic repeats (CRISPRs), RNA editing, RNA interference technology, or a combination thereof.

In some embodiments of the present invention, the BCL11a enhancer (a nucleic acid sequence influencing, for example, enhancing the expression or function of BCL11a) is destroyed by genetic modification (for example, a method selected from the group consisting of: ZFN, TALEN, CRISPR, RNA editing or RNAi). For the BCL11a enhancer, see, for example, Bauer et al., Science, Vol. 342, 2013, pp. 253-257. An example of the BCL11a enhancer is the nucleic acid sequence between exon 2 and exon 3 of BCL11a gene (for example, the nucleic acid located at or corresponding to the positions as recorded in hg38: +55:Chr2:60497676-60498941; +58:Chr2:60494251-60495546; +62:Chr2:60490409-60491734). An example of the BCL11a enhancer is: +62 region of the nucleic acid sequence between exon 2 and exon 3 of BCL11a gene. An example of the BCL11a enhancer is: +58 region of the nucleic acid sequence between exon 2 and exon 3 of BCL11a gene (the 150bp sequence at 58K site of BCL11A gene: ctgccagtcctcttctaccccacccacgcccccaccctaatcagaggccaaacccttcctggagcctgtgataaaagcaactgttagcttgcacta gactagcttcaaagttgtattgaccctggtgtgttatgtctaagagtagatgcc (SEQ ID NO: 2). In some embodiments, the BCL11a enhancer is: +55 region of the nucleic acid sequence between exon 2 and exon 3 of BCL11a gene.

In some embodiments of the above method, BCL11A function is reduced by modifying the BCL11A gene in the region of positions 60495197-60495346 on human chromosome 2. In some embodiments of the above method, BCL11A function is reduced through modifying the CD34-positive HSPCs cells by CRISPR/Cas technology. In some embodiments, the BCL11A function inhibitor is a protein or nucleic acid molecule inhibiting the transcription and/or expression of a gene in the region of positions 60495197-60495346 on human chromosome 2. In some embodiments, the BCL11A function inhibitor is a protein or nucleic acid molecule interfering, inhibiting, or destroying the transcription and/or expression of a gene in the region of positions 60495197-60495346 on human chromosome 2. In some embodiments, the BCL11A function inhibitor is nuclease (such as ZFN and TALEN), peptide nucleic acid, antisense RNA, siRNA, miRNA interfering, inhibiting, or destroying the transcription and/or expression of a gene in the region of positions 60495197-60495346 on human chromosome 2. In some embodiments of the above method, gene editing technology is used to destroy the BCL11A genomic region of positions 60495197-60495346 on human chromosome 2 in the hematopoietic stem cell so as to reduce the BCL11A function. In some embodiments of the above method, the gene editing technology is zinc finger nuclease-based gene editing technology, TALEN gene editing technology or CRISPR/Cas gene editing technology, RNA editing technology, or RNAi technology. In some embodiments of the above method, the gene editing technology is CRISPR/Cas9 gene editing technology. In some embodiments of the above method, the target nucleotide sequence of BCL11A genome is complementary to any sequence selected from the group consisting of: SEQ ID NOs: 3-25.

In some embodiments of the above method, a sgRNA comprising a sequence selected from any one of SEQ ID NOs: 3-25 is introduced into the CD34-positive hematopoietic stem cells/progenitor cells to edit the BCL11A gene, thereby reducing BCL11A function. In some embodiments of the above method, the sgRNA is modified by 2'-O-methyl analog and/or internucleotide 3'-thio. In some embodiments of the above method, the chemical modification is 2'-O-methyl analog modification of one, two and/or three bases before the 5'-end, and/or the last base of the 3'-end of the sgRNA. In some embodiments of the above method, the sgRNA and Cas9-encoding nucleotides are co-introduced into the CD34-positive hematopoietic stem cells/progenitor cells. In some embodiments of the above method, the sgRNA and Cas9-encoding nucleotides are co-introduced into the hematopoietic stem cells by electroporation. In some embodiments of the above method, the electroporation conditions are 200-600V, 0.5ms-2ms.

Cell "differentiation" refers to a process in which cells from the same source gradually produce cell populations with different morphological structures and functional characteristics.

The "differentiation" from hematopoietic stem cells to erythrocytes comprises hematopoietic stem cell stage, erythroid progenitor cell stage, proliferation and differentiation stage of erythroid precursor cells (primary erythrocyte to late erythrocyte), proliferation and maturation process of reticulocytes, and the stage of releasing reticulocytes from peripheral blood to mature into erythrocytes. Hematopoietic stem cell stage: it is currently known that hematopoietic stem cells mainly exist in bone marrow, spleen, liver and other hematopoietic tissues, and a small amount of them also circulates in the peripheral blood. Erythroid progenitor cell stage: in this stage cells are a cell population between hematopoietic stem cells and erythroid precursor cells. Hematopoietic stem cells differentiate into erythroid progenitor cells under the influence of hematopoietic microenvironment of bone marrow. The hematopoietic microenvironment comprises microvascular system, nervous system, and hematopoietic stroma, etc. The differentiation of hematopoietic stem cells is specifically affected and influenced by humoral factors and cytokines. Erythroid precursor cell stage: including primitive erythrocytes, early young erythrocytes, intermediate young erythrocytes, late young erythrocytes, and reticulocytes, to reach mature erythrocytes.

The process of cell maturation is a process in which hemoglobin increases and the activity of nuclear decreases. As the cells mature, the content of hemoglobin in nucleated erythrocytes continues to increase, and the content of RNA continues to decrease. The increase of hemoglobin in erythrocytes makes the nucleus lose activity, and no longer synthesize DNA or RNA. Experiments have confirmed that, this is because hemoglobin enters the nucleus through the pores of nuclear membrane and acts on nucleohistones, resulting in inactivation of chromosomes and promoting nuclear condensation. A late young erythrocyte has lost the ability to continue dividing, later its nucleus is concentrated and escaped to be swallowed by mononuclear macrophage, or to be fragmented and dissolved in the spleen, and thus it becomes a reticulocyte without nuclei. Hemoglobin is no longer synthesized at the stage of mature erythrocytes. According to the theory that the increase of intracellular hemoglobin concentration will cause the cell nucleus to lose activity, the number of divisions of erythrocytes during maturation and the final size of the cell are associated with the speed of hemoglobin synthesis. As the cells mature, the diameter of erythroid cells gradually decreases, and the cell volume gradually decreases. This is because some organelles (such as mitochondria, Golgi apparatus, polyribosomes) for synthesizing hemoglobin, matrix proteins and various enzymes in the cell gradually decrease, and the organelles also gradually degenerate and disappear. Gene activity is dominated by the expression of globin during the process of erythrocyte maturation. Globin only accounts for 0.1% of protein in primitive erythrocyte, and reaches 95% until reticulocyte stage. It is known that the synthesis of adult globin is mainly HbA (α2β2), a small amount of HbA2 (α2δ2) and HbF (α2γ2). The proliferation time of erythroid cells may be estimated by DNA synthesis ability of cell proliferation cycle labeled by radionuclide; the proliferation time of primitive erythrocytes is about 20 hours, that of early young erythrocytes is about 16 hours, and that of intermediate young erythrocytes is 25-30 hours, that of late young erythrocytes do not have the ability to synthesize DNA and are non-proliferative cells. Therefore, normal erythroid precursor cells are generated from the bone marrow, and it takes about 3-5 days to proliferate and differentiate until the new reticulocytes escape from the bone marrow. The reticulocytes then stay in the spleen for 1-2 days, continuing to mature and change the lipid composition of the membrane, and then entering the blood circulation.

"The ability of a desired level of γ-globin or fetal hemoglobin (HbF)" means that compared with the level of γ-globin or fetal hemoglobin produced by unmodified EV cells after differentiation, the level of γ-globin or fetal hemoglobin produced by modified EV cells after differentiation increased by, for example, at least 0.2 times, 0.3 times, 0.4 times, 0.5 times, 1.0 times, 1.5 times, 2.0 times, 2.5 times, 3.0 times, 3.5 times, 4.0 times, 4.5 times, 5 times, 5.5 times, 6.0 times or more. For example, in some embodiments of the present application, compared with unmodified CD34-positive HSPCs, the modified CD34-positive HSPCs produce 120%, 130%, 140%, 150% %, 200%, 250%, 300%, 350%, 400%, 450%, 500%, 550%, 600%, or higher level of γ-globin or fetal hemoglobin.

In the above embodiment, evaluating the ability of a first population of the modified CD34-positive HSPCs to produce a desired level of γ-globin or fetal hemoglobin (HbF) after differentiation refers to: evaluating whether the level of γ-globin or fetal hemoglobin (HbF) produced by the modified CD34-positive HSPCs with reduced BCL11A function after differentiation is increased by at least about 12%, 13%, 14%, 15%, 16%, 17%, 18%, 19%, 20%, 21%, 22%, 23%, 24%, 25%, 26%, 27%, 28%, 29%, 30%, 31% , 32%, 33%, 34%, 35%, 36%, 37%, 38%, 39%, 40%, 50%, 55%, 60%, 65%, 70%, 75%, 80%, 85% %, 90%, 95%, 100%, or 120% than the level of γ-globin or fetal hemoglobin (HbF) produced by the unmodified CD34-positive HSPCs after differentiation.

In some embodiments of the above method, the desired level of fetal hemoglobin is more than 6.5 g/dL, 7.0 g/dL, 7.5 g/dL, 8.0 g/dL, 8.5 g/dL, 9.0 g/dL, 9.5g/dL, 10g/dL, 10.5g/dL, or 11.0g/dL peripheral blood, or higher level.

In some embodiments of the above method, as for the desired level of γ-globin, the modified CD34-positive HSPCs produces more than about 50%, 60%, 70%, 80%, 90%, 100%, 120%, 150%, 200%, 250%, 300%, 350%, 400%, 450%, 500%, 550%, 600%, or higher level of γ-globulin mRNA as compared with the unmodified CD34-positive HSPCs.

The above γ-globulin or fetal hemoglobin (HbF) level may be detected, for example, by detecting γ-globulin mRNA or hemoglobin (HbF), after the CD34-positive HSPCs differentiating to a stage in which annucleated erythrocytes accounts for more than 10% (e.g., 15%, 20%, 30%, 40%, 50%, 60%, 70%, 80%, 90%, or 95%) of the total number of the differentiated cell population, thereby comparing whether the level of γ-globin or fetal hemoglobin (HbF) produced by the modified CD34-positive HSPCs with reduced BCL11A function after differentiation is increased by at least about 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 50%, 55%, 60%, 65%, 70%, 75%, 80%, 85% %, 90%, 95%, 100%, or 120% than the level of γ-globin or fetal hemoglobin (HbF) produced by the unmodified CD34-positive HSPCs after differentiation.

In some embodiments, the modified CD34-positive HSPCs with reduced BCL11A function reduce BCL11A function by modifying the BCL11A gene in the region of positions 60495197-60495346 on human chromosome 2 (e.g., genetic modification by CRISPR/Cas technology, comprising introducing sgRNA comprising a sequence selected from any one of SEQ ID NOs: 3-25 into the CD34-positive hematopoietic stem cells/progenitor cells to edit the BCL11A gene).

In some embodiments of the above method, the ability of the modified EV cells to produce a desired level of γ-globin or fetal hemoglobin (HbF) after differentiation is evaluated in the evaluation step comprising: 1) culturing the modified EV cells under conditions allowing differentiation to obtain an erythrocyte population; and 2) determining the level of γ-globulin or fetal hemoglobin (HbF) produced by the erythrocytes.

In some embodiments of the above method, the ability of the modified EV cells to produce a desired level of γ-globin or fetal hemoglobin (HbF) after differentiation is evaluated in the evaluation step comprising: determining the mRNA level of γ-globulin. In some embodiments of the above method, the ability of the modified EV cells to produce a desired level of γ-globin or fetal hemoglobin (HbF) after differentiation is evaluated in the evaluation step comprising: determining the protein level of fetal hemoglobin (HbF). In some embodiments of the above method, the individual has not undergone CD34-positive HSPCs cell mobilization or pretreatment prior to the evaluation step.

In some embodiments, the CD34-positive HSPCs cell mobilization comprises administering GCSF and/or Mozobil^{™} (Genzyme) or other hematopoietic stem cell mobilizer to the subject.

In some embodiments of the above method, the evaluation step is repeated at least once (e.g., 2, 3, 4, 5 or more times) prior to the treatment step.

The isolated TR cells may be modified by a method identical to or different from the method for modifying the modified isolated EV cells. In some embodiments of the above method, the isolated TR cells are genetically modified. In some embodiments of the above method, in some embodiments of the above method, the method comprises genetically modifying the isolated TR cells. In some embodiments of the above method, the genetic modification comprises genetically modifying the isolated TR cells by any method selected from the group consisting of: zinc finger nuclease (ZFN), transcription activator-like effector nuclease (TALEN), and clustered regularly interspaced short palindromic repeats (CRISPRs), or a combination thereof.

In some embodiments of the present invention, the BCL11a enhancer (a nucleic acid sequence influencing, for example, enhancing the expression or function of BCL11a) in isolated EV cells or TR cells is destroyed by genetic modification (for example, a method selected from the group consisting of: ZFN, TALEN, CRISPR, RNA editing or RNAi). For the BCL11a enhancer, see, for example, Bauer et al., Science, Vol. 342, 2013, pp. 253-257. An example of the BCL11a enhancer is the nucleic acid sequence between exon 2 and exon 3 of BCL11a gene (for example, the nucleic acid located at or corresponding to the positions as recorded in hg38: +55:Chr2:60497676-60498941; +58:Chr2:60494251-60495546; +62:Chr2:60490409-60491734). An example of the BCL11a enhancer is: +62 region of the nucleic acid sequence between exon 2 and exon 3 of BCL11a gene. An example of the BCL11a enhancer is: +58 region of the nucleic acid sequence between exon 2 and exon 3 of BCL11a gene. In some embodiments, the BCL11a enhancer is: +55 region of the nucleic acid sequence between exon 2 and exon 3 of BCL11a gene.

In some embodiments of the above method, BCL11A function is reduced by modifying the BCL11A gene in the region of positions 60495197-60495346 on human chromosome 2. In some embodiments of the above method, BCL11A function is reduced through modifying the CD34-positive HSPCs cells by CRISPR/Cas technology. In some embodiments, the BCL11A function inhibitor is a protein or nucleic acid molecule inhibiting the transcription and/or expression of a gene in the region of positions 60495197-60495346 on human chromosome 2. In some embodiments, the BCL11A function inhibitor is a protein or nucleic acid molecule interfering, inhibiting, or destroying the transcription and/or expression of a gene in the region of positions 60495197-60495346 on human chromosome 2. In some embodiments, the BCL11A function inhibitor is nuclease (such as ZFN and TALEN), peptide nucleic acid, antisense RNA, siRNA, miRNA interfering, inhibiting, or destroying the transcription and/or expression of a gene in the region of positions 60495197-60495346 on human chromosome 2.

In some embodiments of the above method, gene editing technology is used to destroy the BCL11A genomic region of positions 60495197-60495346 on human chromosome 2 in the hematopoietic stem cell so as to reduce the BCL11A function. In some embodiments of the above method, the gene editing technology is zinc finger nuclease-based gene editing technology, TALEN gene editing technology or CRISPR/Cas gene editing technology, RNA editing technology, or RNAi technology. In some embodiments of the above method, the gene editing technology is CRISPR/Cas9 gene editing technology. In some embodiments of the above method, the target nucleotide sequence of BCL11A genome is complementary to any sequence selected from the group consisting of: SEQ ID NOs: 3-25.

The following table lists the genomic sequence positions on human chromosome 2 targeted by the sgRNAs represented by SEQ ID NOs: 3-25, and the cleavage site for the Cas9 cleavage triggered by each sgRNA.

| Name | Position on the human genome sequence | Cleavage site |
|---|---|---|
| Enhancer-7 of BCL11A | chr2:60495203-60495222 | chr2:60495219 |
| Enhancer-8 of BCL11A | chr2:60495208-60495227 | chr2:60495224 |
| Enhancer-9 of BCL11A | chr2:60495217-60495236 | chr2:60495233 |
| Enhancer-10 of BCL11A | chr2:60495218-60495237 | chr2:60495234 |
| Enhancer-11 of BCL11A | chr2:60495219-60495238 | chr2:60495235 |
| Enhancer-14 of BCL11A | chr2:60495221-60495240 | chr2:60495223 |
| Enhancer-12 of BCL11A | chr2:60495222-60495241 | chr2:60495238 |
| Enhancer-13 of BCL11A | chr2:60495223-60495242 | chr2:60495239 |
| Enhancer-15 of BCL11A | chr2:60495228-60495247 | chr2:60495244 |
| Enhancer-16 of BCL11A | chr2:60495229-60495248 | chr2:60495245 |
| Enhancer-17 of BCL11A | chr2:60495230-60495249 | chr2:60495246 |
| Enhancer-18 of BCL11A | chr2:60495231-60495250 | chr2:60495247 |
| Enhancer-19 of BCL11A | chr2:60495234-60495253 | chr2:60495250 |
| Enhancer-20 of BCL11A | chr2:60495235-60495254 | chr2:60495251 |
| Enhancer-2 of BCL11A | chr2:60495236-60495255 | chr2:60495238 |
| Enhancer-1 of BCL11A | chr2:60495247-60495266 | chr2:60495263 |
| Enhancer-6 of BCL11A | chr2:60495252-60495271 | chr2:60495268 |
| Enhancer-5 of BCL11A | chr2:60495253-60495272 | chr2:60495269 |
| Enhancer-4 of BCL11A | chr2:60495257-60495276 | chr2:60495273 |
| Enhancer-3 of BCL11A | chr2:60495264-60495283 | chr2:60495280 |
| Enhancer-22 of BCL11A | chr2:60495299-60495318 | chr2:60495301 |
| Enhancer-23 of BCL11A | chr2:60495319-60495338 | chr2:60495335 |
| Enhancer-21 of BCL11A | chr2:60495320-60495339 | chr2:60495336 |

SEQ ID NO: 3: a sgRNA named as enhancer-1 of BCL11A (sometimes abbreviated as enhancer-1); sgRNA coding sequence:
cacaggctccaggaagggtt

SEQ ID NO: 4: a sgRNA named as enhancer-2 of BCL11A (sometimes abbreviated as enhancer-2); sgRNA coding sequence:
atcagaggccaaacccttcc

SEQ ID NO: 5: a sgRNA named as enhancer-3 of BCL11A (sometimes abbreviated as enhancer-3); sgRNA coding sequence:
Ctaacagttgcttttatcac

SEQ ID NO: 6: a sgRNA named as enhancer-4 of BCL11A (sometimes abbreviated as enhancer-4); sgRNA coding sequence:
ttgcttttatcacaggctcc

SEQ ID NO: 7: a sgRNA named as enhancer-5 of BCL11A (sometimes abbreviated as enhancer-5); sgRNA coding sequence:
ttttatcacaggctccagga

SEQ ID NO: 8: a sgRNA named as enhancer-6 of BCL11A (sometimes abbreviated as enhancer-6); sgRNA coding sequence:
tttatcacaggctccaggaa

SEQ ID NO: 9: a sgRNA named as enhancer-7 of BCL11A (sometimes abbreviated as enhancer-7); sgRNA coding sequence:
tgggtggggtagaagaggac

SEQ ID NO: 10: a sgRNA named as enhancer-8 of BCL11A (sometimes abbreviated as enhancer-8); sgRNA coding sequence:
gggcgtgggtggggtagaag

SEQ ID NO: 11: a sgRNA named as enhancer-9 of BCL11A (sometimes abbreviated as enhancer-9); sgRNA coding sequence:
ttagggtgggggcgtgggtg

SEQ ID NO: 12: a sgRNA named as enhancer-10 of BCL11A (sometimes abbreviated as enhancer-10); sgRNA coding sequence:
attagggtgggggcgtgggt

SEQ ID NO: 13: a sgRNA named as enhancer-11 of BCL11A (sometimes abbreviated as enhancer-11); sgRNA coding sequence:
gattagggtgggggcgtggg

SEQ ID NO: 14: a sgRNA named as enhancer-12 of BCL11A (sometimes abbreviated as enhancer-12); sgRNA coding sequence:
tctgattagggtgggggcgt

SEQ ID NO: 15: a sgRNA named as enhancer-13 of BCL11A (sometimes abbreviated as enhancer-13); sgRNA coding sequence:
ctctgattagggtgggggcg

SEQ ID NO: 16: a sgRNA named as enhancer-14 of BCL11A (sometimes abbreviated as enhancer-14); sgRNA coding sequence:
cacgcccccaccctaatcag

SEQ ID NO: 17: a sgRNA named as enhancer-15 of BCL11A (sometimes abbreviated as enhancer-15); sgRNA coding sequence:
ttggcctctgattagggtgg

SEQ ID NO: 18: a sgRNA named as enhancer-16 of BCL11A (sometimes abbreviated as enhancer-16); sgRNA coding sequence:
tttggcctctgattagggtg

SEQ ID NO: 19: a sgRNA named as enhancer-17 of BCL11A (sometimes abbreviated as enhancer-17); sgRNA coding sequence:
gtttggcctctgattagggt

SEQ ID NO: 20: a sgRNA named as enhancer-18 of BCL11A (sometimes abbreviated as enhancer-18); sgRNA coding sequence:
ggtttggcctctgattaggg

SEQ ID NO: 21: a sgRNA named as enhancer-19 of BCL11A (sometimes abbreviated as enhancer-19); sgRNA coding sequence:
aagggtttggcctctgatta

SEQ ID NO: 22: a sgRNA named as enhancer-20 of BCL11A (sometimes abbreviated as enhancer-20); sgRNA coding sequence:
gaagggtttggcctctgatt

SEQ ID NO: 23: a sgRNA named as enhancer-21 of BCL11A (sometimes abbreviated as enhancer-21); sgRNA coding sequence:
actcttagacataacacacc

SEQ ID NO: 24: a sgRNA named as enhancer-22 of BCL11A (sometimes abbreviated as enhancer-22); sgRNA coding sequence:
cttcaaagttgtattgaccc

SEQ ID NO: 25: a sgRNA named as enhancer-23 of BCL11A (sometimes abbreviated as enhancer-23); sgRNA coding sequence:
ctcttagacataacacacca

After analyzing the cleavage sites of the above 23 sgRNAs, it is found that the cleavage sites for the Cas9 cleavage triggered by these sgRNAs are concentrated in the genomic region of positions 60495219-60495336 of BCL11A gene.

Generally speaking, the guide sequence in sgRNA is any polynucleotide sequence that has sufficient complementarity with the target sequence to hybridize with the target sequence and direct the sequence-specific binding of the CRISPR complex to the target sequence. In some embodiments, when an appropriate alignment algorithm is used for optimal alignment, the degree of complementarity between the guide sequence and its corresponding target sequence is about or greater than about 80%, 85%, 90%, 95%, 97.5%, 99% or more. The optimal alignment may be determined by any appropriate algorithm for aligning sequences, and the non-limiting examples include: Smith-Waterman algorithm, Needleman-Wimsch algorithm, Burrows-Wheeler Transform-based algorithm (such as Burrows Wheeler Aligner), ClustalW, Clustai X, BLAT, Novoalign (Novocraft Technologies), ELAND (Illumina, San Diego, CA), SOAP (available at: soap.genomics.org.cn) and Maq (available at: maq.sourceforge.net). In some embodiments, the length of the guide sequence may be about or greater than about 10, 11, 12, 13, 14, 15, 16, 17, 18, 19, 20, 21, 22, 23, 24, 25, 26, 27, 28, 29, 30, 35, 40, 45, 50, 55, 60, 65, 70, 75 or more nucleotides. In some embodiments, the length of the guide sequence is less than about 75, 70, 65, 60, 55, 50, 45, 40, 35, 30, 25, 20, 15, 12 or fewer nucleotides. The ability of the guide sequence to direct the sequence-specific binding of the CRISPR complex to the target sequence may be assessed by any appropriate assay method. For example, the components of the CRISPR system (comprising the guide sequence to be tested) sufficient to form a CRISPR complex may be provided for a host cell with a corresponding target sequence, for example, it may be performed by transfecting with a vector encoding the CRISPR sequence components, and then evaluating the preferential cleavage within the target sequence (as determined by Surveyor as described herein). Similarly, the cleavage of the target polynucleotide sequence may be conducted in the test tube by providing components of the target sequence, the CRISPR complex (comprising the guide sequence to be tested and the control guide sequence different from the guide sequence), then comparing the rate of binding or cleavage of the tested sequence and control guide sequence on the target sequence, thereby performing the evaluation. Other assay methods known to those skilled in the art may also be used to perform the above detection and evaluation.

In some embodiments, the sgRNA used in the gene editing process is chemically modified. "Chemically modified sgRNA" refers to special chemical modification of sgRNA, such as 2'-O-methyl analog modification at the 3 bases of its 5'- and 3'-ends and/or internucleotide 3'-thio modification.

The chemically modified sgRNA has at least the following two advantages. Firstly, since sgRNA is a single-stranded form of RNA with a very short half-life, after entering the cell it will be degraded rapidly (maximum 12 hours), while it takes at least 48hours for Cas9 protein to bind sgRNA to perform gene editing. Therefore, chemically modified sgRNA is adopted, after entering the cell it is stably expressed, and after being combined with the Cas9 protein, gene editing may be efficiently performed on the genome to produce Indels. Secondly, unmodified sgRNA has poor ability to penetrate cell membranes and cannot effectively enter cells or tissues to perform corresponding functions. The ability of chemically modified sgRNA to penetrate cell membranes is usually enhanced. In the present invention, chemical modification methods commonly used in the art may be used, as long as they may improve the stability of sgRNA (extending the half-life) and enhance the ability to enter the cell membrane. In addition to the specific chemical modifications used in the Examples, other modification methods may also be used, for example, the chemical modification methods reported in the following literatures: Deleavey GF1, Damha MJ. Designing chemically modified oligonucleotides for targeted gene silencing. Chem Biol. 2012 Aug 24; 19(8):937-54; and Hendel et al., Chemically modified guide RNAs enhance CRISPR-Cas genome editing in human primary cells. Nat Biotechnol. 2015 Sep; 33(9):985-989.

In some embodiments, the sgRNA and/or Cas9-encoding nucleotides (such as mRNA) are introduced into the hematopoietic stem cells by electrotransduction, for example, introducing into the hematopoietic stem cells under the electroporation conditions of: 250-360V, 0.5-1ms; 250-300V, 0.5-1ms; 250V-1ms; 250V 2ms; 300V 0.5ms; 300V 1ms; 360V 0.5ms; or 360Vlms. In some embodiments, the sgRNA and Cas9-encoding nucleotides are co-introduced into the hematopoietic stem cells by electroporation. In some embodiments, the sgRNA is introduced into the hematopoietic stem cells expressing Cas9 by electrotransduction.

In some embodiments, the Cas9-encoding nucleotides are mRNA, such as mRNA comprising ARCA cap. In some embodiments, the Cas9-encoding nucleotides are in a viral vector, such as a lentiviral vector. In some embodiments, the Cas9-encoding nucleotides comprise the sequence represented by SEQ ID NO:26. In some embodiments, the sgRNA and the Cas9-encoding nucleotides are in the same vector.

In some embodiments of the above method, sgRNA comprising any sequence selected from the group consisting of: SEQ ID NOs: 3-25 is introduced into the CD34-positive hematopoietic stem cell/progenitor cell to edit the BCL11A gene, thereby reducing BCL11A function. In some embodiments of the above method, the sgRNA is modified by 2'-O-methyl analog and/or internucleotide 3'-thio. In some embodiments of the above method, the chemical modification is 2'-O-methyl analog modification at the first one, the first two and/or the first three bases of the 5'-end and/or at the last base of the 3'-end of the sgRNA. In some embodiments of the above method, the sgRNA and Cas9-encoding nucleotides are co-introduced into the CD34-positive hematopoietic stem cell/progenitor cell. In some embodiments of the above method, sgRNA and Cas9-encoding nucleotides are co-introduced into the hematopoietic stem cell by electroporation. In some embodiments of the above method, the electroporation conditions are 200-600V, 0.5-2ms.

In some embodiments of the above method, mobilizing CD34-positive HSPCs in the treatment step comprises: treating the individual with granulocyte colony stimulating factor (GCSF) and/or plerixafor.

In some embodiments of the above method, the treatment step further comprises: pretreating the individual prior to administering the modified TR cells.

In some embodiments, the pretreatment comprises chemotherapy, monoclonal antibody therapy, or systemic radiation. In some embodiments, the chemotherapy comprises administering to the individual one or more chemotherapeutic agents selected from the group consisting of: busulfan, cyclophosphamide, and fludarabine.

In some embodiments of the above method, the modified EV cell population is cultured in vitro to further differentiate into precursor cells of erythrocytes or mature erythrocytes expressing γ-globin or fetal hemoglobin.

In some embodiments, the precursor cells of erythrocytes are precursor cells capable of expressing γ-globin or fetal hemoglobin prior to becoming mature erythrocytes.

In some embodiments of the above method, a medium for erythroid expansion and differentiation of hematopoietic stem cells is used to perform erythroid expansion and differentiation of the hematopoietic stem cells on the modified EV cell population, wherein the medium for erythroid expansion and differentiation of hematopoietic stem cells comprises: a basal medium and a composition of growth factors, and wherein the composition of growth factors comprises: stem cell growth factor (SCF); interleukin-3 (IL-3) and erythropoietin (EPO). In some embodiments, also included is the step of performing erythroid differentiation and denucleation of hematopoietic stem cells by using a medium for erythroid differentiation and denucleation, wherein the medium for erythroid differentiation and denucleation comprises: a basal medium, growth factors, and antagonists and/or inhibitors of progesterone receptor and glucocorticoid receptor. In some embodiments, the growth factors in the medium for erythroid differentiation and denucleation comprise erythropoietin (EPO), and the antagonists and/or inhibitors of the progesterone receptor and glucocorticoid receptor are any one or two or more selected from the group consisting of the following compounds (I)-(IV):

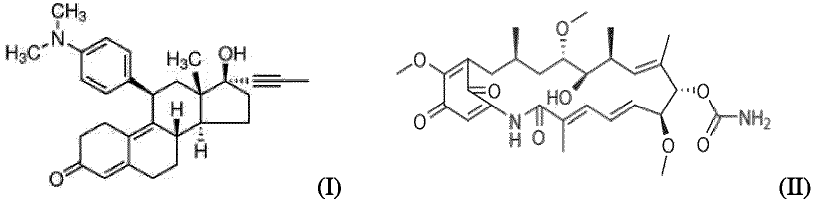

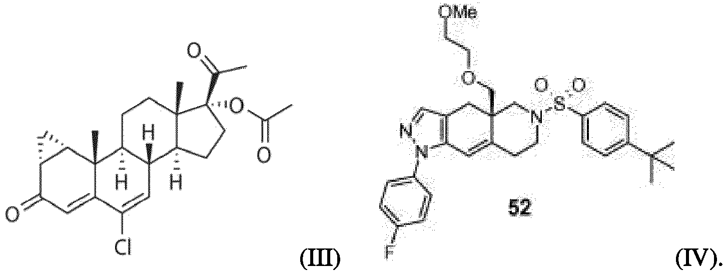

In some embodiments, the medium for erythroid expansion and differentiation of hematopoietic stem cells comprises: a basal medium and growth factor additives, wherein the basal medium may be selected from any serum-free basal medium, such as STEMSPAN^{™} SFEM II (STEM CELLS TECHNOLOGY Inc.), or IMDM (Iscove's Modified Dulbecco's Medium), optionally supplemented with ITS (Thermofisher), L-gulutamin (Thermofisher), vitamin C and/or bovine serum albumin; wherein the growth factor additive is any one selected from the group consisting of: IL-3, SCF and EPO, or a combination thereof.

In some embodiments of the above method, precursor cells of erythrocytes, such as erythroblasts, nucleated erythrocytes, young erythrocytes and reticulocytes are obtained after 5-10 days, e.g., 6-10 days, or 7-10 days of expansion and differentiation of the modified EV cell population. The precursor cells of erythrocytes are then subjected to about 4-14 days, e.g., about 4, 5, 6, 7, 8, 9, 10, 11, 12, 13, 14 days of differentiation (or differentiation and denucleation) treatment to obtain mature erythrocytes.

In some embodiments, the step of differentiating the modified EV cell population into mature erythrocytes that produce a desired level of γ-globin or fetal hemoglobin (HbF) may include:
a) obtaining CD34-positive hematopoietic stem cells/progenitor cells (isolated CD34-positive HSPCs) from human peripheral blood (with or without mobilization of the hematopoietic stem cells in bone marrow) or bone marrow,
b) genetically modifying the above CD34-positive HSPCs to reduce BCL11A function;
c) adding additional growth factors in a serum-free medium (SFME) to perform 5-10 days, e.g., 5 days, 6 days, 7 days, 8 days, 9 days, and 10 days of expansion and differentiation, thereby differentiating the HSPCs into precursor cells of erythrocytes;
d) adding additional growth factors in a serum-free medium (SFME) to perform about 4-14 days (e.g., about 4, 5, 6, 7, 8, 9, 10, 11, 12, 13, 14 or more days) of differentiation, thereby obtaining mature erythrocytes. In some embodiments, the medium for erythroid differentiation and denucleation of hematopoietic stem cells comprises: a basal medium, growth factors, and chemical small molecule additives, wherein the basal medium is a serum-free basal medium, such as STEMSPAN^{™} SFEM II (STEM CELLS Technology Inc.), IMDM (Iscove's Modified Dulbecco's Medium), optionally added with ITS (Thermofisher), L-gulutamin (Thermofisher), vitamin C and/or bovine serum albumin, growth factors, and chemical small molecule additives comprising EPO, human transferrin and/or chemical small molecule mifepristone.

The modified CD34-positive HSPCs (EV cell population) differentiate to produce γ-globin mRNA or hemoglobin (HbF) which may be detected by conventional methods in the art. For example, when the modified CD34-positive HSPCs (EV cell population) differentiate to a stage in which annucleated erythrocytes accounts for more than 10% (e.g., 15%, 20%, 30%, 40%, 50%, 60%, 70%, 80%, 90%, 95%) of the total number of the differentiated cell population, the level of the resulting γ-globin or fetal hemoglobin (HbF) may be detected by conventional methods in the art; comparing whether the level of γ-globin or fetal hemoglobin (HbF) produced by the modified CD34-positive HSPCs with reduced BCL11A function after differentiation is higher than the level of γ-globin or fetal hemoglobin (HbF) produced by the unmodified CD34-positive HSPCs after differentiation, if it is increased by at least about 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 50%, 55%, 60%, 65%, 70%, 75%, 80%, 85% %, 90%, 95%, 100%, or 120%, indicating that the method of the present invention may be used for treatment. Any commonly used basal medium may be used in the above medium for erythroid expansion and differentiation of hematopoietic stem cells, and medium for erythroid differentiation and denucleation of hematopoietic stem cells, such as STEMSPAN^{™} SFEM II (purchased from STEM CELL TECHONOLOGIES); e.g., IMDM, DF12, Knockout DMEM, RPMI 1640, Alpha MEM, DMEM, etc. purchased from Thermo Fisher. In addition, other components may be further added to these basal media as needed, for example, ITS (i.e., mainly comprising insulin, human transferrin, and selenium), L-glutamine, vitamin C, and bovine serum albumin may be added. For example, ITS, 2mM L-glutamine, 10-50µg/ml vitamin C and 0.5-5 mass% BSA (bovine serum albumin) may be added to the IMDM medium. In addition, the above DF12 may be added with the same concentration of ITS, L-glutamine, vitamin C and bovine serum albumin. Knockout DMEM may be added with the same concentration of ITS, L-glutamine, vitamin C and bovine serum albumin; RPMI 1640 may be added with the same concentration of ITS, L-glutamine, vitamin C and bovine serum albumin; Alpha MEM may be added with the same concentration of ITS, L-glutamine, vitamin C and bovine serum albumin; DMEM may also be added with the same concentration of ITS, L-glutamine, vitamin C and bovine serum albumin. Herein, the concentration of additional ITS in various basal media may be: 0.1 mg/ml of insulin concentration, 0.0055 mg/ml of human transferrin, and 6.7×10⁻⁶ mg/ml of selenium. In addition, the concentration of each component of the additional ITS may also be adjusted according to actual needs. ITS may be purchased from Thermofisher and adjusted to the appropriate final working concentration as needed.

In some embodiments of the above method, the modified TR cell population is not proliferated ex vivo or in vitro prior to being administered to the individual. In a particular embodiment, the modified TR cells may be washed to remove the treatment reagents, and administered to a patient without proliferating them ex vivo. In some embodiments, the modified TR cells are administered to a patient prior to occurrence of any significant ex vivo cell division, or prior to the time required for any significant ex vivo cell division. In some embodiments, after modification the modified TR cells are cultured for one or more days prior to being administered to an individual. In some embodiments, after modification the modified TR cells are administered to a patient within 2, 4, 6, 12, 24, or 48 hours.

In some embodiments, the modified TR cells are stored under a freezing condition for at least 24 hours prior to being administered to the individual. In some embodiments, the modified TR cells are cultured for one or more days prior to being stored under a freezing condition. The modified TR cells may be cultured in a serum-free basal medium supplemented with cytokines maintaining cell viability for one or more days (e.g., 1-3 days), the cytokine is, for example, SCF (stem cell factor), TPO (thrombopoietin), FLT-3L (FMS-like tyrosine kinase-3 ligand), or IL-6 (interleukin-6). For example, the cells may be cultured in stem cell growth medium (CellGenix) for one or more days (e.g., 1-3 days).

In some embodiments, the isolated TR cells are cultured for one or more days prior to modification, and then the isolated TR cells are modified.

In some embodiments of the above method, the hemoglobinopathy is selected from the group consisting of: sickle cell disease, sickle cell trait, hemoglobin C disease, hemoglobin C trait, hemoglobin S/C disease, hemoglobin D disease, hemoglobin E disease, thalassemia, hemoglobin-related disorder with increased oxygen affinity, hemoglobin-related disorder with decreased oxygen affinity, unstable hemoglobin disease and methemoglobinemia. In some embodiments, the hemoglobinopathy are selected from the group consisting of: β-thalassemia and sickle cell anemia. In some embodiments, the hemoglobinopathy is β⁰ or β⁺ thalassemia.

In some embodiments of the above method, the individual is a human. In some embodiments of the above method, the treatment step is performed immediately following the evaluation step.

As used herein, the term "treatment" refers to obtaining the desired pharmacological and/or physiological effects, comprising but not limited to: achieving improvement or elimination of disease symptoms. The effect may be prophylactic, manifesting as complete or partial prevention of the disease or its symptoms; and/or the effect may be therapeutic, manifesting as improvement or elimination of symptoms, or providing partial or complete cure of the disease or the adverse effects due to the disease. As used herein, "treatment" comprises any treatment of a disease in a mammal, especially human, comprising: (a) preventing the onset of a disease in an individual; (b) inhibiting a disease, i.e., preventing its development; (c) alleviating a disease, e.g., causing remission of the disease, for example, complete or partial elimination of disease symptoms; and (d) returning the individual to a pre-disease state, for example, rebuilding the blood system. In this application, "treatment" does not necessarily mean the complete eradication or cure of a disease or disease state, or its related symptoms, and it encompasses any minimal improvement or alleviation of any one or more measurable manifestations of the disease or disease state. In the specific methods described above, treatment comprises improvement in hematopoietic reconstitution or survival of the individual.

### Exemplary Embodiments:

1. A method for treating hemoglobinopathy in an individual, which comprises:
   a) an evaluation step comprising: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells"); and
   b) a treatment step comprising: administering a second population of the modified CD34-positive HSPCs to the individual, wherein the modified CD34-positive HSPCs are derived from the individual and are modified to reduce BCL11A function ("modified TR cells").
2. A method for treating hemoglobinopathy in an individual, which comprises a treatment step comprising: administering a second population of the modified CD34-positive HSPCs to the individual, wherein the modified CD34-positive HSPCs are derived from the individual and are modified to reduce BCL11A function ("modified TR cells"), and
   wherein the individual is selected for treatment based on the functional evaluation from an evaluation step which comprises: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells").
3. A method for selecting an individual suffering from hemoglobinopathy for treatment with a second population of the modified CD34-positive HSPCs which are derived from the individual and are modified to reduce BCL11A function ("modified TR cells"), wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), wherein if the first population of the modified CD34-positive HSPCs produce a desired level of γ-globin or fetal hemoglobin (HbF), the individual is selected for treatment.
4. A method for determining whether an individual suffering from hemoglobinopathy is suitable for treating with a second population of the modified CD34-positive HSPCs ("modified TR cells") derived from the individual and modified to reduce BCL11A function, wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), and wherein if the first population of the modified CD34-positive HSPCs produce a desired level of γ-globin or fetal hemoglobin (HbF), the individual is suitable for treatment.
5.A method for determining whether an individual suffering from hemoglobinopathy is unsuitable for treating with a second population of the modified CD34-positive HSPCs ("modified TR cells") derived from the individual and modified to reduce BCL11A function, wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), and wherein if the first population of the modified CD34-positive HSPCs do not produce a desired level of γ-globin or fetal hemoglobin (HbF), the individual is not suitable for treating with the modified TR cells.
6. The method according to any one of embodiments 1-5, wherein the evaluation step comprises:
   a) isolating CD34-positive HSPCs from the bone marrow or peripheral blood sample of the individual to obtain an isolated CD34-positive HSPCs population ("isolated EV cells");
   b) modifying the isolated EV cells to obtain a first population of the modified CD34-positive HSPCs cells with reduced BCL11A function ("modified EV cells"); and
   c) evaluating the ability of the modified EV cells to produce a desired level of γ-globin or fetal hemoglobin (HbF) after differentiation.
7. The method according to any one of embodiments 1-6, wherein the treatment step comprises:
   a) mobilize the CD34-positive HSPCs in the bone marrow of the individual to increase the amount of CD34-positive HSPCs in the peripheral blood;
   b) isolating CD34-positive HSPCs from the peripheral blood of the individual to obtain an isolated CD34-positive HSPCs population ("isolated TR cells"),
   c) modifying the isolated TR cells to obtain a second population of the modified CD34-positive HSPCs with reduced BCL11A function ("modified TR cells"); and
   d) administering an effective amount of the modified TR cells to the individual.
8. A method for treating hemoglobinopathy in an individual, which comprises:
   1) an evaluation step, comprising:
      a) isolating CD34-positive HSPCs from the bone marrow or peripheral blood sample of the individual to obtain an isolated CD34-positive HSPCs population ("isolated EV cells");
      b) modifying the isolated EV cells to obtain a first population of the modified CD34-positive HSPCs cells with reduced BCL11A function ("modified EV cells"); and
      c) evaluating the ability of the modified EV cells to produce a desired level of γ-globin or fetal hemoglobin (HbF) after differentiation; and
   2) a treatment step, comprising:
      a) mobilizing the CD34-positive HSPCs in the individual to the peripheral blood;
      b) isolating CD34-positive HSPCs from the peripheral blood of the individual to obtain an isolated CD34-positive HSPCs population ("isolated TR cells"),
      c) modifying the isolated TR cells to obtain a second population of the modified CD34-positive HSPCs with reduced BCL11A function ("modified TR cells"); and
      d) administering an effective amount of the modified TR cells to the individual.
9. The method according to any one of embodiments 1-8, wherein the modified EV cells are modified by genetic modification.
10. The method according to any one of embodiments 6-8, wherein modifying the isolated EV cells comprises genetically modifying the isolated EV cells.
11. The method according to embodiment 9 or 10, wherein the isolated EV cells are genetically modified by a technology selected from the group consisting of: zinc finger nuclease (ZFN), transcription activator-like effector nuclease (TALEN), and clustered regularly interspaced short palindromic repeats (CRISPRs), RNA editing, RNA interference.
12. The method according to any one of embodiments 1-11, wherein the ability of the modified EV cells to produce a desired level of γ-globin or fetal hemoglobin (HbF) after differentiation is evaluated in the evaluation step comprising: 1) culturing the modified EV cells under conditions allowing differentiation to obtain an erythrocyte population; and 2) determining the level of γ-globulin or fetal hemoglobin (HbF) produced by the erythrocytes.
13. The method according to any one of embodiments 1-12, wherein the ability of the modified EV cells to produce a desired level of γ-globin or fetal hemoglobin (HbF) after differentiation is evaluated in the evaluation step comprising: determining the mRNA level of γ-globulin.
14. The method according to any one of embodiments 1-12, wherein the ability of the modified EV cells to produce a desired level of γ-globin or fetal hemoglobin (HbF) after differentiation is evaluated in the evaluation step comprising: determining the protein level of fetal hemoglobin (HbF).
15. The method according to any one of embodiments 1-14, wherein the individual has not undergone mobilization or pretreatment prior to the evaluation step.
16. The method according to any one of embodiments 1-15, wherein the evaluation step is repeated at least once prior to the treatment step.
17. The method according to any one of embodiments 1-16, wherein the modified TR cells are modified by genetic modification.
18. The method according to any one of embodiments 7-16, wherein modifying the isolated TR cells comprises genetically modifying the isolated TR cells.
19. The method according to embodiment 17 or 18, wherein the isolated TR cells are genetically modified by a technology selected from the group consisting of: zinc finger nuclease (ZFN), transcription activator-like effector nuclease (TALEN), and clustered regularly interspaced short palindromic repeats (CRISPRs), RNA editing, RNA interference.
20. The method according to any one of embodiments 7-19, wherein mobilizing CD34-positive HSPCs in the treatment step comprises: treating the individual with granulocyte colony stimulating factor (GCSF) and/or plerixafor.
21. The method according to any one of embodiments 1-20, wherein the treatment step further comprises: pretreating the individual prior to administering the modified TR cells.
22. The method according to embodiment 21, wherein the pretreatment comprises chemotherapy, monoclonal antibody therapy, or systemic radiation.
23. The method according to embodiment 22, wherein the pretreatment comprises chemotherapy.
24. The method according to embodiment 23, wherein the chemotherapy comprises administering to the individual one or more chemotherapeutic agents selected from the group consisting of: busulfan, cyclophosphamide, and fludarabine.
25. The method according to any one of embodiments 7-24, wherein the isolated TR cells are cultured for one or more days prior to modification.
26. The method according to any one of embodiments 7-25, wherein the modified TR cells are cultured for one or more days prior to being administered to the individual.
27. The method according to any one of embodiments 1-26, wherein the modified TR cells are stored under a freezing condition for at least 24 hours prior to administering the modified TR cells to the individual.
28. The method according to embodiment 27, wherein the modified TR cells are cultured for one or more days prior to being stored under a freezing condition.
29. The method according to any one of embodiments 1-28, wherein the hemoglobinopathy is a disease selected from the group consisting of: sickle cell disease, sickle cell trait, hemoglobin C disease, hemoglobin C trait, hemoglobin S/C disease, hemoglobin D disease, hemoglobin E disease, thalassemia, hemoglobin-related disorder with increased oxygen affinity, hemoglobin-related disorder with decreased oxygen affinity, unstable hemoglobin disease and methemoglobinemia.
30. The method according to embodiment 29, wherein the hemoglobinopathy is selected from the group consisting of: β-thalassemia and sickle cell anemia.
31. The method according to embodiment 30, wherein the hemoglobinopathy is β⁰ or β⁺ thalassemia.
32. The method according to any one of embodiments 1-31, wherein the individual is a human.
33. The method according to any one of embodiments 1-32, wherein the treatment step is performed immediately following the evaluation step.

In some particular embodiments of all the above embodiments, evaluating the ability of a first population of the modified CD34-positive HSPCs to produce a desired level of γ-globin or fetal hemoglobin (HbF) after differentiation refers to: evaluating whether the level of γ-globin or fetal hemoglobin (HbF) produced by the modified CD34-positive HSPCs with reduced function of BCL11A after differentiation is increased by at least about 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 50%, 55%, 60%, 65%, 70%, 75%, 80%, 85%, 90%, 95%, 100%, 120%, 150%, 200%, 250%, 300%, 350%, 400%, 450%, 500%, 550%, or 600% than the level of γ-globin or fetal hemoglobin (HbF) produced by the unmodified CD34-positive HSPCs after differentiation.

### This application also involves the following embodiments

1. Use of the modified TR cells in the preparation of a medicament for use in a method for treating hemoglobinopathy in an individual, the method comprises:
   a) an evaluation step comprising: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells"); and
   b) a treatment step comprising: administering a second population of the modified CD34-positive HSPCs to the individual, wherein the modified CD34-positive HSPCs are derived from the individual and are modified to reduce BCL11A function ("modified TR cells").
2. Use of the modified TR cells in the preparation of a medicament for use in a method for treating hemoglobinopathy in an individual, the method comprises a treatment step comprising: administering a second population of the modified CD34-positive HSPCs to the individual, wherein the modified CD34-positive HSPCs are derived from the individual and are modified to reduce BCL11A function ("modified TR cells"), and
   wherein the individual is selected for treatment based on the functional evaluation from an evaluation step which comprises: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells").
3. Use of the modified EV cells in the preparation of a product for use in a method for determining whether an individual suffering from hemoglobinopathy is suitable or unsuitable for treating with a second population of the modified CD34-positive HSPCs ("modified TR cells") derived from the individual and modified to reduce BCL11A function, wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), and where if the first population of the modified CD34-positive HSPCs produce a desired level of γ-globin or fetal hemoglobin (HbF), the individual is suitable for treatment; wherein if the first population of the modified CD34-positive HSPCs do not produce a desired level of γ-globin or fetal hemoglobin (HbF), the individual is not suitable for treating with the modified TR cells.
4. The use according to any one of embodiments 1-3, wherein the evaluation step comprises:
   a) isolating CD34-positive HSPCs from the bone marrow or peripheral blood sample of the individual to obtain an isolated CD34-positive HSPCs population ("isolated EV cells");
   b) modifying the isolated EV cells to obtain a first population of the modified CD34-positive HSPCs cells with reduced BCL11A function ("modified EV cells"); and
   c) evaluating the ability of the modified EV cells to produce a desired level of γ-globin or fetal hemoglobin (HbF) after differentiation.
5. The use according to any one of embodiments 1-4, wherein the treatment step comprises:
   a) mobilize the CD34-positive HSPCs in the bone marrow of the individual to increase the amount of CD34-positive HSPCs in the peripheral blood;
   b) isolating CD34-positive HSPCs from the peripheral blood of the individual to obtain an isolated CD34-positive HSPCs population ("isolated TR cells"),
   c) modifying the isolated TR cells to obtain a second population of the modified CD34-positive HSPCs with reduced BCL11A function ("modified TR cells"); and
   d) administering an effective amount of the modified TR cells to the individual.
6. The use according to any one of embodiments 1-5,
   1) the evaluation step comprises:
      a) isolating CD34-positive HSPCs from the bone marrow or peripheral blood sample of the individual to obtain an isolated CD34-positive HSPCs population ("isolated EV cells");
      b) modifying the isolated EV cells to obtain a first population of the modified CD34-positive HSPCs cells with reduced BCL11A function ("modified EV cells"); and
      c) evaluating the ability of the modified EV cells to produce a desired level of γ-globin or fetal hemoglobin (HbF) after differentiation; and
   2) the treatment step comprises:
      a) mobilizing the CD34-positive HSPCs in the individual to the peripheral blood;
      b) isolating CD34-positive HSPCs from the peripheral blood of the individual to obtain an isolated CD34-positive HSPCs population ("isolated TR cells"),
      c) modifying the isolated TR cells to obtain a second population of the modified CD34-positive HSPCs with reduced BCL11A function ("modified TR cells"); and
      d) administering an effective amount of the modified TR cells to the individual.
7. The use according to any one of embodiments 1-6, wherein the modified EV cells are modified by genetic modification.
8. The use according to any one of embodiments 4-6, wherein modifying the isolated EV cells comprises genetically modifying the isolated EV cells.
9. The use according to embodiment 7 or 8, wherein the isolated EV cells are genetically modified by a technology selected from the group consisting of: zinc finger nuclease (ZFN), transcription activator-like effector nuclease (TALEN), and clustered regularly interspaced short palindromic repeats (CRISPRs), RNA editing, RNA interference.
10. The use according to any one of embodiments 1-9, wherein the ability of the modified EV cells to produce a desired level of γ-globin or fetal hemoglobin (HbF) after differentiation is evaluated in the evaluation step comprising: 1) culturing the modified EV cells under conditions allowing differentiation to obtain an erythrocyte population; and 2) determining the level of γ-globulin or fetal hemoglobin (HbF) produced by the erythrocytes.
11. The use according to any one of embodiments 1-10, wherein the ability of the modified EV cells to produce a desired level of γ-globin or fetal hemoglobin (HbF) after differentiation is evaluated in the evaluation step comprising: determining the mRNA level of γ-globulin.
12. The use according to any one of embodiments 1-10, wherein the ability of the modified EV cells to produce a desired level of γ-globin or fetal hemoglobin (HbF) after differentiation is evaluated in the evaluation step comprising: determining the protein level of fetal hemoglobin (HbF).
13. The use according to any one of embodiments 1-12, wherein the individual has not undergone mobilization or pretreatment prior to the evaluation step.
14. The use according to any one of embodiments 1-13, wherein the evaluation step is repeated at least once prior to the treatment step.
15. The use according to any one of embodiments 1-14, wherein the modified TR cells are modified by genetic modification.
16. The use according to any one of embodiments 5-14, wherein modifying the isolated TR cells comprises genetically modifying the isolated TR cells.
17. The use according to embodiment 15 or 16, wherein the isolated TR cells are genetically modified by a technology selected from the group consisting of: zinc finger nuclease (ZFN), transcription activator-like effector nuclease (TALEN), and clustered regularly interspaced short palindromic repeats (CRISPRs), RNA editing, RNA interference.
18. The use according to any one of embodiments 5-17, wherein mobilizing CD34-positive HSPCs in the treatment step comprises: treating the individual with granulocyte colony stimulating factor (GCSF) and/or plerixafor.
19. The use according to any one of embodiments 1-18, wherein the treatment step further comprises: pretreating the individual prior to administering the modified TR cells.
20. The use according to embodiment 19, wherein the pretreatment comprises chemotherapy, monoclonal antibody therapy, or systemic radiation.
21. The use according to embodiment 20, wherein the pretreatment comprises chemotherapy.
22. The use according to embodiment 21, wherein the chemotherapy comprises administering to the individual one or more chemotherapeutic agents selected from the group consisting of: busulfan, cyclophosphamide, and fludarabine.
23. The use according to any one of embodiments 5-22, wherein the isolated TR cells are cultured for one or more days prior to modification.
24. The use according to any one of embodiments 5-23, wherein the modified TR cells are cultured for one or more days prior to being administered to the individual.
25. The use according to any one of embodiments 1-24, wherein the modified TR cells are stored under a freezing condition for at least 24 hours prior to administering the modified TR cells to the individual.
26. The use according to embodiment 25, wherein the modified TR cells are cultured for one or more days prior to being stored under a freezing condition.
27. The use according to any one of embodiments 1-26, wherein the hemoglobinopathy is a disease selected from the group consisting of: sickle cell disease, sickle cell trait, hemoglobin C disease, hemoglobin C trait, hemoglobin S/C disease, hemoglobin D disease, hemoglobin E disease, thalassemia, hemoglobin-related disorder with increased oxygen affinity, hemoglobin-related disorder with decreased oxygen affinity, unstable hemoglobin disease and methemoglobinemia.
28. The use according to embodiment 27, wherein the hemoglobinopathy is selected from the group consisting of: β-thalassemia and sickle cell anemia.
29. The use according to embodiment 28, wherein the hemoglobinopathy is β⁰ or β⁺ thalassemia.
30. The use according to any one of embodiments 1-29, wherein the individual is a human.
31. The use according to any one of embodiments 1-30, wherein the treatment step is performed immediately following the evaluation step.

In some particular embodiments of all the above embodiments, evaluating the ability of a first population of the modified CD34-positive HSPCs to produce a desired level of γ-globin or fetal hemoglobin (HbF) after differentiation refers to: evaluating whether the level of γ-globin or fetal hemoglobin (HbF) produced by the modified CD34-positive HSPCs with reduced BCL11A function after differentiation is increased by at least about 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 50%, 55%, 60%, 65%, 70%, 75%, 80%, 85%, 90%, 95%, 100%, 120%, 150%, 200%, 250%, 300%, 350%, 400%, 450%, 500%, 550%, or 600% than the level of γ-globin or fetal hemoglobin (HbF) produced by the unmodified CD34-positive HSPCs after differentiation.

### Example

### Example 1: Gene Editing of the BCL11A Enhancer of Hematopoietic Stem Cells

This example involves gene editing of BCL11A erythroid enhancer sites of CD34-positive hematopoietic stem cells derived from mobilized peripheral blood of a thalassemia patient and healthy donor by CRISPR/Cas9 system.

"CRISPR RGEN TOOLS" software is used to design sgRNA targeting BCL11A(+58) site, and two chemically modified sgRNAs are synthesized. The sequence information is as follows: sgRNA-1: ctaacagttgcttttatcac (SEQ ID NO: 5); sgRNA-2: atcagaggccaaacccttcc (SEQ ID NO: 4). The coding sequence information of Cas9 mRNA is as follows:

gacaagaagtacagcatcggcctggacatcggcaccaactctgtgggctgggccgtgatcaccgacgagtacaaggtgcccagcaagaaatt
caaggtgctgggcaacaccgaccggcacagcatcaagaagaacctgatcggagccctgctgttcgacagcggcgaaacagccgaggccac
ccggctgaagagaaccgccagaagaagatacaccagacggaagaaccggatctgctatctgcaagagatcttcagcaacgagatggccaag
gtggacgacagcttcttccacagactggaagagtccttcctggtggaagaggataagaagcacgagcggcaccccatcttcggcaacatcgtg
gacgaggtggcctaccacgagaagtaccccaccatctaccacctgagaaagaaactggtggacagcaccgacaaggccgacctgcggctga
tctatctggccctggcccacatgatcaagttccggggccacttcctgatcgagggcgacctgaaccccgacaacagcgacgtggacaagctgtt
catccagctggtgcagacctacaaccagctgttcgaggaaaaccccatcaacgccagcggcgtggacgccaaggccatcctgtctgccagac
tgagcaagagcagacggctggaaaatctgatcgcccagctgcccggcgagaagaagaatggcctgttcggcaacctgattgccctgagcctg
ggcctgacccccaacttcaagagcaacttcgacctggccgaggatgccaaactgcagctgagcaaggacacctacgacgacgacctggaca
acctgctggcccagatcggcgaccagtacgccgacctgtttctggccgccaagaacctgtccgacgccatcctgctgagcgacatcctgagag
tgaacaccgagatcaccaaggcccccctgagcgcctctatgatcaagagatacgacgagcaccaccaggacctgaccctgctgaaagctctc
gtgcggcagcagctgcctgagaagtacaaagagattttcttcgaccagagcaagaacggctacgccggctacattgacggcggagccagcca
ggaagagttctacaagttcatcaagcccatcctggaaaagatggacggcaccgaggaactgctcgtgaagctgaacagagaggacctgctgc

ggaagcagcggaccttcgacaacggcagcatccccccaccagatccacctgggagagctgcacgccattctgcggcggcaggaagatttttac
ccattcctgaaggacaaccgggaaaagatcgagaagatcctgaccttccgcatcccctactacgtgggccctctggccaggggaaacagcag
attcgcctggatgaccagaaagagcgaggaaaccatcaccccctggaacttcgaggaagtggtggacaagggcgcttccgcccagagcttca
tcgagcggatgaccaacttcgataagaacctgcccaacgagaaggtgctgcccaagcacagcctgctgtacgagtacttcaccgtgtataacg
agctgaccaaagtgaaatacgtgaccgagggaatgagaaagcccgccttcctgagcggcgagcagaaaaaggccatcgtggacctgctgttc
aagaccaaccggaaagtgaccgtgaagcagctgaaagaggactacttcaagaaaatcgagtgcttcgactccgtggaaatctccggcgtgga
agatcggttcaacgcctccctgggcacataccacgatctgctgaaaattatcaaggacaaggacttcctggacaatgaggaaaacgaggacatt
ctggaagatatcgtgctgaccctgacactgtttgaggacagagagatgatcgaggaacggctgaaaacctatgcccacctgttcgacgacaaa
gtgatgaagcagctgaagcggcggagatacaccggctggggcaggctgagccggaagctgatcaacggcatccgggacaagcagtccgg
caagacaatcctggatttcctgaagtccgacggcttcgccaacagaaacttcatgcagctgatccacgacgacagcctgacctttaaagaggac
atccagaaagcccaggtgtccggccagggcgatagcctgcacgagcacattgccaatctggccggcagccccgccattaagaagggcatcct
gcagacagtgaaggtggtggacgagctcgtgaaagtgatgggccggcacaagcccgagaacatcgtgatcgaaatggccagagagaacca
gaccacccagaagggacagaagaacagccgcgagagaatgaagcggatcgaagagggcatcaaagagctgggcagccagatcctgaaa
gaacaccccgtggaaaacacccagctgcagaacgagaagctgtacctgtactacctgcagaatgggcgggatatgtacgtggaccaggaact
ggacatcaaccggctgtccgactacgatgtggaccatatcgtgcctcagagctttctgaaggacgactccatcgacaacaaggtgctgaccaga
agcgacaagaaccggggcaagagcgacaacgtgccctccgaagaggtcgtgaagaagatgaagaactactggcggcagctgctgaacgcc
aagctgattacccagagaaagttcgacaatctgaccaaggccgagagaggcggcctgagcgaactggataaggccggcttcatcaagagac
agctggtggaaacccggcagatcacaaagcacgtggcacagatcctggactcccggatgaacactaagtacgacgagaatgacaagctgatc
cgggaagtgaaagtgatcaccctgaagtccaagctggtgtccgatttccggaaggatttccagttttacaaagtgcgcgagatcaacaactacca
ccacgcccacgacgcctacctgaacgccgtcgtgggaaccgccctgatcaaaaagtaccctaagctggaaagcgagttcgtgtacggcgact
acaaggtgtacgacgtgcggaagatgatcgccaagagcgagcaggaaatcggcaaggctaccgccaagtacttcttctacagcaacatcatg
aactttttcaagaccgagattaccctggccaacggcgagatccggaagcggcctctgatcgagacaaacggcgaaaccggggagatcgtgtg
ggataagggccgggattttgccaccgtgcggaaagtgctgagcatgccccaagtgaatatcgtgaaaaagaccgaggtgcagacaggcggct
tcagcaaagagtctatcctgcccaagaggaacagcgataagctgatcgccagaaagaaggactgggaccctaagaagtacggcggcttcgac
agccccaccgtggcctattctgtgctggtggtggccaaagtggaaaagggcaagtccaagaaactgaagagtgtgaaagagctgctggggat
caccatcatggaaagaagcagcttcgagaagaatcccatcgactttctggaagccaagggctacaaagaagtgaaaaaggacctgatcatcaa
gctgcctaagtactccctgttcgagctggaaaacggccggaagagaatgctggcctctgccggcgaactgcagaagggaaacgaactggccc
tgccctccaaatatgtgaacttcctgtacctggccagccactatgagaagctgaagggctcccccgaggataatgagcagaaacagctgtttgtg
gaacagcacaagcactacctggacgagatcatcgagcagatcagcgagttctccaagagagtgatcctggccgacgctaatctggacaaagt
gctgtccgcctacaacaagcaccgggataagcccatcagagagcaggccgagaatatcatccacctgtttaccctgaccaatctgggagcccc

tgccgccttcaagtactttgacaccaccatcgaccggaagaggtacaccagcaccaaagaggtgctggacgccaccctgatccaccagagcat
caccggcctgtacgagacacggatcgacctgtctcagctgggaggcgac. (SEQ ID NO: 1)

The chemical synthesis of sgRNA means that the first three bases at the 5'-end and the last three bases at the 3'-end of sgRNA are modified with 2'-O-methyl analog modification and internucleotide 3'-thio modification. As shown in the following chemical formula, the left side is the chemically modified sgRNA, and the right side is the unmodified sgRNA. Both Cas9 mRNA and sgRNA are purchased from Trilink Biotechnologies, USA.

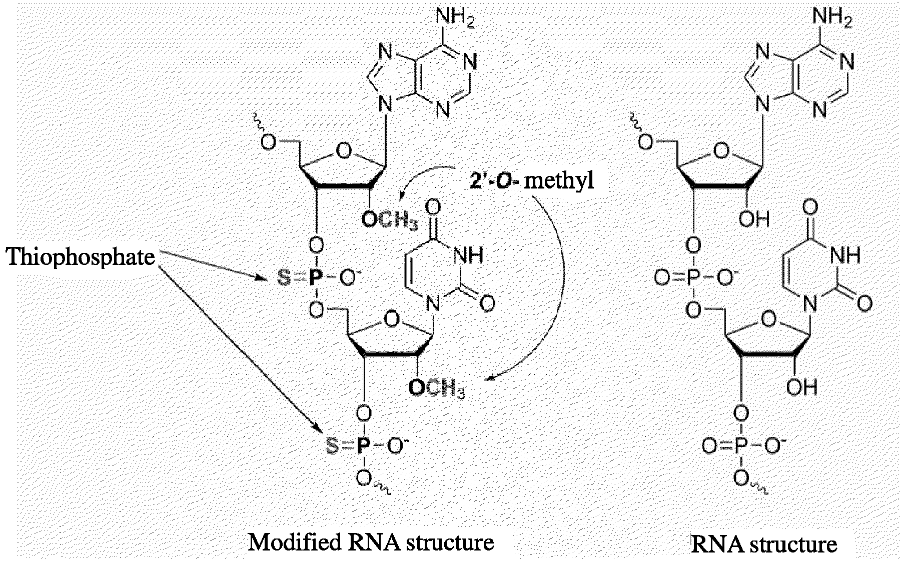

We isolated CD34-positive hematopoietic stem cells from the mobilized peripheral blood of 5 patients with thalassemia and 2 healthy donors (samples of the mobilized peripheral blood are from Nanfang-Chunfu Children's Institute of Hematology and Oncology). Selecting the electroporation conditions of "300V 1ms" in the BTX ECM830 electroporator, the synthesized Cas9 mRNA and sgRNA-1 and sgRNA-2 synthesized by the chemical modification are respectively introduced into CD34-positive hematopoietic stem cells from 5 patients and 2 healthy donors by electroporation; 4 days after electroporation, the genome of the CD34-positive hematopoietic stem cells is extracted, and a fragment with a total length of 903 bp (453 bp on the left side and 450 bp on the right side of the sgRNA cleavage site) is selected for amplification and Sanger sequencing.

Forward primer: cacctcagcagaaacaaagttatc (SEQ ID NO: 26)

Reverse primer: gggaagctccaaactctcaa (SEQ ID NO: 27)

The statistical analysis on the efficiency of generating Indels is performed based on the sequencing results by "Synthego ICE Analysis" online software, wherein the "Synthego ICE Analysis" online software is online software for analyzing the efficiency of Indels. The efficiency of double-peak mutations caused by Indels is analyzed based on the first-generation sequencing results, referring to the following website:
https://www.synthego.com/products/bioinformatics/crispr-analysis.

Referring to Fig. 1, the results show that the two sgRNAs synthesized in this example successfully perform gene-editing of CD34-positive hematopoietic stem cells derived from mobilized peripheral blood of different thalassemia patients and healthy donors; wherein the gene editing efficiency of sgRNA-1 is 30-70%, and the efficiency of Indels varies significantly between different donors. By contrast it is significantly different that, sgRNA-2 may edit the cells more efficiently and stably to produce Indels, thereby destroying the BCL11A erythroid enhancer; as for the 5 patient donors and 2 healthy donors, the efficiency of gene editing reaches 60-80%.

### Example 2: Expression of γ-globin mRNA and Erythroid Differentiation-related Proteins

This experiment verifies the expression of γ-globin (HBG gene) mRNA of the genetically edited hematopoietic stem cells after differentiation, wherein the hematopoietic stem cells are derived from the mobilized peripheral blood of thalassemia patients and healthy donors.

### 2.1 Erythrocyte differentiation

Selecting the electroporation conditions of "300v 1ms", Cas9 mRNA and sgRNA-1, Cas9 mRNA and sgRNA-2 are respectively introduced into the hematopoietic stem cells from the mobilized peripheral blood of 5 thalassemia patients and 2 healthy donors by electroporation. The cells in control group do not undergo the electroporation step. After that, the erythrocyte differentiation experiment is performed through a differentiation protocol of the following "two-step method".

In the "two-step method" differentiation, firstly a medium for erythroid expansion and differentiation of hematopoietic stem cells is used to induce the CD34+ HSPCs to differentiate into erythroid progenitor cells for differentiation, and then a medium for erythroid differentiation and denucleation of hematopoietic stem cells is used to induce the erythroid progenitor cells to differentiate into mature erythrocytes.

The basal medium in the medium for erythroid expansion and differentiation of hematopoietic stem cells is StemSpan^{™} SFEM II; the growth factors are: 50-200 ng/ml SCF, 10-100 ng/ml IL-3, and 1-10U EPO/ml; culture conditions: CD34+ HSPCs are cultured in the medium for erythroid expansion and differentiation of hematopoietic stem cells at a cell density of 1.0×10⁵ cells/ml, 7 days after differentiation, the CD34+ HSPCs differentiate into erythroid progenitor cells.

The basal medium in the medium for erythroid differentiation and denucleation of hematopoietic stem cells is StemSpan^{™} SFEM II; the growth factors are: 1-10U EPO, 100-1000 µg/ml human transferrin, and the chemical small molecule is 0.5-10 µm mifepristone. The erythroid progenitor cells cultured in the previous step are used to differentiate in the medium for erythroid differentiation and denucleation of hematopoietic stem cells at a cell density of 1.0×10⁶ cells/ml, 11 days later the erythroid progenitor cells differentiate and mature into mature erythrocytes.

We detect the expression of cell surface membrane proteins CD71 and CD235a expressed by erythrocytes, and the ratio of CD71 and CD235a double positive is used to indicate the efficiency of differentiation of CD34+ HSPCs into erythrocytes, as shown in Fig. 2. The experimental group comprises: all of the sgRNA-1 and sgRNA-2, and the control group efficiently differentiate into erythrocytes, the expression ratios of CD71 and CD235a are both above about 80%, and the differentiation efficiency is high, indicating that the gene editing effects of sgRNA-1 and sgRNA-2 are both relatively good.

### 2.2 Detection of the mRNA expression of γ-globin (HBG) gene in erythrocytes differentiated from hematopoietic stem cells

The mRNA of the cells is extracted from the mature erythrocytes differentiated from the hematopoietic stem cells in Example 2.1, and is reversely transcribed into cDNA, and the mRNA expression of HBG gene is detected by fluorescent quantitative PCR. As shown in Fig. 3.

The experimental results indicate that:
1) In the CD34-positive hematopoietic stem cells from the mobilized samples of the thalassemia patients and healthy donors, gene-editing of the erythroid enhancer site of BCL11A is performed to relieve the inhibition of BCL11A on γ-globin (HBG) and fetal hemoglobin HbF; in the cells of the two experimental groups of sgRNA-1 and sgRNA-2, the mRNA expression levels of γ-globin (HBG) are increased.
2) particularly:
   a. for patient patient 1, sgRNA-1 is increased by about 4.2 times, and sgRNA-2 is increased by about 3.8 times.
   b. for patient patient 2, sgRNA-1 is increased by about 1.8 times, and sgRNA-2 is increased by about 2 times.
   c. for patient patient 3, sgRNA-1 is increased about 2.1 times, and sgRNA-2 is increased about 3.1 times.
   d. for patient patient 4, sgRNA-1 is increased by about 4 times, and sgRNA-2 is increased by about 5.8 times.
   e. for patient 5, sgRNA-1 is increased by about 1.6 times, and sgRNA-2 is increased by about 2.2 times.
   f. for healthy donor 1, sgRNA-1 is increased about 3.0 times, and sgRNA-2 is increased about 6.1 times.
   g. for healthy donor 2, sgRNA-1 is increased by about 1.7 times, and sgRNA-2 is increased by about 1.7 times.

Combining the above data, it may be found that whether it is sgRNA-1 or sgRNA-2, in different donors (patients and healthy donors), although gene-editing of the erythroid enhancer site of BCL11A is performed to relieve the inhibition of BCL11A on γ-globin (HBG) and fetal hemoglobin HbF, the increased level of γ-globin (HBG) varies from donor to donor.

In addition, although the increased levels of γ-globin (HBG) caused by sgRNA-1 and sgRNA-2 are different, as compared with donors with a low increased level of γ-globin (HBG), in the donors with higher increased levels of γ-globin (HBG), sgRNA-1 and sgRNA-2 have the same tendency, with a high degree of consistency and stability. For example, for patient donor 1, both sgRNA-1 and sgRNA-2 are increased by about 4 times; while for patient donor 2, both sgRNA-1 and sgRNA-2 are only increased by about 2 times; for healthy donor 1, both sgRNA-1 and sgRNA-2 are increased by 3.0-6.0 times, while for healthy donor 2, both sgRNA-1 and sgRNA-2 are increased by less than 2 times.

### Example 3: Multi-batch verification experiment for gene editing efficiency detection

### 3.1 Mobilized peripheral blood from healthy donors

This experiment involves a multi-batch verification experiment for efficient gene-editing of the BCL11A erythroid enhancer site of CD34-positive hematopoietic stem cells from mobilized peripheral blood of 2 healthy donors by CRISPR/Cas9 system.

It can be seen from the results of Example 1 and Example 2 that, Cas9 mRNA and sgRNA-2 may be used for efficient and stable gene editing of BCL11A erythroid enhancer site, and the gene editing efficiency is 60-80% which is higher than sgRNA-1; moreover, after releasing the inhibition of BCL11A on γ-globin (HBG) and fetal hemoglobin HbF, increased mRNA level of γ-globin (HBG) caused by sgRNA-2 may higher than that of sgRNA-1. Therefore, in this example, sgRNA-2 is selected as the preferred target for subsequent experiments.

Selecting the electroporation conditions of "300V 1ms" in the BTX ECM830 electroporator, the synthesized Cas9 mRNA and sgRNA-2 synthesized by chemical modification are respectively introduced into CD34-positive hematopoietic stem cells from 2 healthy donors by electroporation; 4 days after electroporation, the genome of the CD34-positive hematopoietic stem cells is extracted, and a fragment with a total length of 903 bp (about 450 bp on the left side and right side of the sgRNA cleavage site) is selected for amplification and Sanger sequencing. As for the sequencing primers and method for analyzing the efficiency of gene editing, please refer to Example 1. The experimental results are shown in Fig. 4A

The experimental results show that, under the electroporation conditions, the sgRNA-2 synthesized in this example successfully and efficiently performs gene-editing of the CD34-positive hematopoietic stem cells from mobilized peripheral blood of different donors, and efficiently produce Indels; in 3 batches of experiments for 2 healthy donors, the gene editing efficiency reaches 70-80%. Wherein, "3 batches of experiments" means that, 1 sample of mobilized peripheral blood is obtained from each healthy donor, and CD34-positive hematopoietic stem cells are isolated, however 3 independent experiments of gene editing are performed for these cells.

### 3.2 Patients with thalassemia mobilize peripheral blood sources

Selecting the electroporation conditions of "300V 1ms" in the BTX ECM830 electroporator, Cas9 mRNA and the sgRNA-2 synthesized by chemical modification are respectively introduced into CD34-positive hematopoietic stem cells from 10 thalassemia patients by electroporation. The control group cells do not undergo the electroporation step. 2 days later, the genome is extracted, and a fragment with a total length of 903 bp (about 450 bp on the left side and right side of the sgRNA cleavage site) is selected for PCR amplification, and the PCR products are used for Sanger sequencing. As for the sequencing primers and method for analyzing the efficiency of gene editing, please refer to Example 1. The experimental results are shown in Fig. 4B.

The experimental results show that, under the electroporation conditions, the sgRNA-2 synthesized in this example successfully and efficiently performs gene-editing of the CD34-positive hematopoietic stem cells from mobilized peripheral blood of different thalassemia patients, and efficiently produce Indels; in multiple batches of experiments for 10 thalassemia patients, the gene editing efficiency reaches 60-80% or more; wherein 1 sample of mobilized peripheral blood is obtained from each of the 10 thalassemia patients, and CD34-positive hematopoietic stem cells are isolated for experiments of gene editing. As for these cells, 3 independent experiments of gene editing are performed for patient donor 1; 2 independent experiments of gene editing are performed respectively for the three patient donors, donor 6, donor 9 and donor 10, while a single gene editing experiment is performed for the remaining patient donors.

### Example 4: Expression of γ-globin mRNA and Fetal Hemoglobin HbF of Hematopoietic

### Stem Cells after Differentiation

This experiment relates to detecting the expression of BCL11A gene and γ-globin mRNA of the genetically edited hematopoietic stem cells after differentiation, wherein the hematopoietic stem cells are derived from the mobilized peripheral blood of healthy donors.

### 4.1 Erythrocyte differentiation

### 4.1.1 Healthy donors

Selecting the electroporation conditions of "300v 1ms", Cas9 mRNA and sgRNA-2 are respectively introduced into the hematopoietic stem cells from the mobilized peripheral blood of 2 healthy donors by electroporation. The cells in control group do not undergo the electroporation step. Three batches of erythrocyte differentiation experiments is performed through a differentiation protocol of the following "two-step method".

The "two-step method" differentiation comprises: firstly performing differentiation in a medium for erythroid expansion and differentiation of hematopoietic stem cells, then performing differentiation in a medium for erythroid differentiation and denucleation of hematopoietic stem cells.

The basal medium in the medium for erythroid expansion and differentiation of hematopoietic stem cells is StemSpan^{™} SFEM II; the growth factors are: 50-200 ng/ml SCF, 10-100 ng/ml IL-3, and 1-10U EPO/ml; culture conditions: the hematopoietic stem cells are cultured in the medium for erythroid expansion and differentiation of hematopoietic stem cells at 1.0×10⁵ cells/ml, and the cell expansion is performed for 7 days.

The basal medium in the medium for erythroid differentiation and denucleation of hematopoietic stem cells is StemSpan^{™} SFEM II; the growth factors are: 1-10U EPO, 100-1000 µg/ml human transferrin, and the chemical small molecule is 0.5-10 µm mifepristone. The cells cultured in the previous step are used to differentiate in the medium for erythroid differentiation and denucleation of hematopoietic stem cells at 1.0×10⁶ cells/ml for 11 days.

We detect the expression of CD71 and CD235a by FACS method, as shown in Fig. 5. The cells in both the experimental group and the control group differentiate into erythrocytes with high efficiency, the expression ratios of CD71 and CD235a are both above about 85%, the differentiation efficiency is high, and the effect is good. Wherein, "3 batches of erythrocyte differentiation experiments" means that, 1 sample of mobilized peripheral blood is obtained from each healthy donor, and CD34-positive hematopoietic stem cells are isolated; while 3 independent experiments of gene editing are performed for these cells, and 3 erythrocyte differentiation experiments are respectively performed.

### 4.1.2 Thalassemia patients

Selecting the electroporation conditions of "300V 1ms", Cas9 mRNA and sgRNA-2 are respectively introduced into hematopoietic stem cells from mobilized peripheral blood of 10 thalassemia patients by electroporation. The control group cells do not undergo the electroporation step. Multiple batches of erythrocyte differentiation experiments are performed by the differentiation protocol of "two-step method" in 4.1.1.

After 18 days of differentiation, the expressions of CD71 and CD235a are detected, as shown in Fig. 6. The cells in both the experimental group and the control group differentiate into erythrocytes with high efficiency. The erythroid differentiation efficiency of different thalassemia patients are different between individuals; the double positive expression ratio of CD71 and CD235a is about 60-95%, indicating that its differentiation efficiency is high and the effect is good. Wherein, 1 sample of mobilized peripheral blood is obtained from each of the 10 thalassemia patients, and CD34-positive hematopoietic stem cells are isolated; wherein the CD34-positive hematopoietic stem cells from patient donor 1 are used to perform 3 independent experiments of gene editing, and 3 independent erythrocyte differentiation experiments; the CD34-positive hematopoietic stem cells from the three patient donors, donor 6, donor 9 and donor 10 are used to respectively perform 2 independent experiments of gene editing, and 2 erythrocyte differentiation experiments; while a single gene editing experiment and a single erythrocyte differentiation experiment are performed for the remaining 6 patient donors.

### 4.2 Detection of mRNA expression of γ-globin and BCL11A gene in erythrocytes differentiated from hematopoietic stem cells of healthy donors

mRNA of the cells extracted from the mature erythrocytes differentiated from hematopoietic stem cells in Example 4.1.1 is reversely transcribed into cDNA, and the mRNA expressions of genes such as BCL11A and HBG are detected by fluorescent quantitative PCR. As shown in Figs. 7-8.

Experimental results show that:
1) for healthy donors 1, the results of the three batches of experiments show that after gene editing of the BCL11A erythroid enhancer of hematopoietic stem cells, the expression of BCL11A gene in the experimental group is down-regulated and is about 40-80% of the expression in the control group, while the gene expression of γ-globin (HBG gene) is significantly increased, and the increased level in the three batches of experiments is about 6-10 times that of the control group, and the results are highly consistent and stable.
2) for healthy donors 2, the results of the three batches of experiments show that after gene editing of the BCL11A erythroid enhancer of hematopoietic stem cells, the expression of BCL11A gene in the experimental group is down-regulated and is about 40-80% of the expression in the control group, while the gene expression of γ-globin (HBG gene) is significantly increased, and the increased level in the three batches of repetitive experiments is about 1.5-3.5 times that of the control group, and the results are highly consistent and stable.

The results of this experiment further prove that, as for different donors, after electroporation of sgRNA and Cas9 mRNA targeting the erythroid enhancer of BCL11A, the down-regulation rate of BCL11A gene expression is 20-60%; while the increased levels of γ-globin expression are significantly different. For healthy donor 1, the expression of γ-globin is increased by 6-10 times; and for healthy donor 2, the expression of γ-globin is increased by 1.5-3.5 times. Herein, "3 batches of erythrocyte differentiation experiments" means that, 1 sample of mobilized peripheral blood is respectively obtained from each healthy donor, and CD34-positive hematopoietic stem cells are isolated; while 3 independent experiments of gene editing are performed for these cells, and 3 erythrocyte differentiation experiments are respectively performed, and the mRNA expression level of BCL11A gene and γ-globin (HBG gene) are detected.

### 4.3 Detection of fetal hemoglobin HbF expression in erythrocytes derived from hematopoietic stem cells of thalassemia patients

The positive expression ratio of fetal hemoglobin HbF in the mature erythrocytes differentiated from the hematopoietic stem cells in Example 4.1.2 are detected by flow cytometry. As shown in Figs. 9A, 9B and 10.

Experimental results show that:
1) compared with the control group without undergoing gene editing, the expression of HbF in erythrocytes differentiated from the CD34-positive hematopoietic stem cells undergoing gene editing is increased.
2) the increased expression levels of HbF are significantly different between different thalassemia patients, wherein in the 3 batches of experiments, the expression levels of HbF for patient donor 1 and patient donor 7 are about 1.3 times that of the control group, and the expression levels of HbF for patient donor 2 are about 2.3 times that of the control group. However, the expression levels of HbF for the same thalassemia patient in different batches of experiments are very similar, wherein in 2 batches of experiments, the expression levels of HbF for patient donor 1 are about 1.3 times that of the control group, the expression levels of HbF for patient donor 9 are about 1.4 times that of the control group, and the expression levels of HbF for patient donor 10 are about 1.5-1.6 times that of the control group.
3) the expression level of HbF for patient donor 1 in the experimental group is 1.29±0.12 times that of the control group. Therefore, we believe that if the expression level of HbF after gene editing is increased by at least about 12% than that of the control group, i.e., the value of one standard deviation, then it is effective for this thalassemia patient.

### Example 5: Treatment of Hemoglobinopathy by Gene Editing of BCL11A Erythroid

### Enhancer

It is not difficult to find from the results of Example 2.2, Example 4.2 and Example 4.3 that, after electroporation of Cas9 mRNA and the same sgRNA for BCL11A erythroid enhancer, the increased expression levels of γ-globin and fetal hemoglobin HbF are significant different for different donors. The main reason is that, as described in the background art of this application, the mechanism of regulating and inhibiting the expression of γ-globin and fetal hemoglobin HbFb in mature erythrocytes is complicated, it involves LCR in the remote regulatory region, and there are also multiple transcription factors such as BCL11A, MYB , KLF1 and others combining to form a complex as a switch for regulating γ-globin and fetal hemoglobin HbF (G. Lettre, et al. PNAS. 2008, 11869-11874; Marina et al, Molecular Therapy. 2017; Megan D, et al. Blood. 2015). It means that the inhibitory effects of BCL11A on γ-globin and fetal hemoglobin HbF are significantly different between individuals.

In addition, although the regulatory mechanism for γ-globin and fetal hemoglobin HbF is complex, for the same individual, from the fetal stage to birth and to adult stage, the regulatory mechanism of γ-globin and fetal hemoglobin HbF is relatively constant, which means that the inhibition degree and effect of BCL11A gene on γ-globin and fetal hemoglobin HbF are basically unchanged (Paikari A, et al. Br J Haematol. 2018; Lettre G, et al. Lancet. 2016; G. Lettre, et al. PNAS .2008,11869-11874; Marina et al, Molecular Therapy. 2017; Megan D, et al. Blood. 2015), and this is further confirmed from the results in Examples 4.2 and 4.3. For the same donor, after gene editing of BCL11A erythroid enhancer site and erythrocyte differentiation, in the multiple batches of experiments for detection and analysis of γ-globin (HBG gene) mRNA expression and fetal hemoglobin HbF, their increased levels are consistent and stable.

Therefore, combining the experimental results and phenomena of this application, the current treatment strategy of gene-editing of BCL11A erythroid enhancers for treating hemoglobinopathy has obvious potential defects, i.e., the prior art does not predict the regulation level and effect of BCL11A on γ-globin and fetal hemoglobin HbF in advance, after a patient is treated with large dose of agent for clearing bone marrow and autologous hematopoietic stem cell transplantation, it is very likely that the therapeutic protocol will be ineffective or the effect will not be significant, and this will bring potentially huge harm to the patient. This application provides an effective prediction method, i.e., the increased levels and effect of γ-globin and fetal hemoglobin HbF in different individuals are different, and the results are consistent and stable; while in different batches of experiments for the hematopoietic stem cells of the same individual, after performing gene editing to down-regulate the expression of BCL11A, the increased levels and effect of γ-globin and fetal hemoglobin HbF are consistent and stable; if the fetal hemoglobin HbF may be increased by at least about 12% after gene editing, the therapeutic protocol is considered to be effective for thalassemia patients .

Based on the experimental results, this application proposes a new method for treating hemoglobinopathy by gene-editing of BCL11A erythroid enhancer, i.e., a therapy of companion diagnosis + gene-editing of autologous hematopoietic stem cells. As shown in Fig. 11.

Companion diagnosis: before treating a patient with hemoglobinopathy by gene-editing of BCL11A erythroid enhancer, we may predict the effectiveness of the therapeutic protocol in advance, i.e., extracting a small amount of bone marrow or peripheral blood from the patient in advance, and isolating the CD34-positive hematopoietic stem cells, then performing gene editing of the BCL11A erythroid enhancer site, and differentiating the genetically edited cells into mature erythrocytes, wherein the expression levels of γ-globin (HBG gene) and fetal hemoglobin HbF are evaluated by a series of indicators, and a patient with higher increased levels of γ-globin and fetal hemoglobin HbF expression is selected as a preferential subject for treatment, thus the probability of the therapeutic protocol being effective is higher. Wherein, if compared with the control group without undergoing gene editing, the expression of fetal hemoglobin HbF in the experimental group is increased by at least about 12%, then a patient receiving treatment may benefit from clinical treatment. Because on the one hand, it is clinically believed that a patient with hemoglobin content of 90g/L may get rid of blood transfusion dependence; if a patient with higher increased levels of γ-globin and fetal hemoglobin HbF expression is selected for treatment, the possibility of getting rid of blood transfusion dependence is greater. On the other hand, although a 90g/L patient may get rid of blood transfusion, mild manifestations of anemia still exist (90-110g/L), and bone development may also be affected (normally, the hemoglobin content range for a man is 120-160g/L, and for a woman is 110-150g/L); if a patient with higher increased levels of γ-globin and fetal hemoglobin HbF expression is selected for treatment, the possibility of complete recovery of the patient is greater. Therefore, the goal of the therapeutic strategy of treating hemoglobinopathy by gene editing of BCL11A erythroid enhancer is to make the total hemoglobin expression of the patient as high as possible, and if it reaches the standard of a normal person, the therapeutic effect is more significant, and the patient benefits more. In addition, only a small amount of bone marrow or peripheral blood is needed to extract in the prediction method developed in this application, and the process is simple. A patient does not need to go through the complicated process of administrating mobilization agent to make the bone marrow produce a large amount of hematopoietic stem cells, and during the screening period the patient will not be injected large dose of agent for clearing bone marrow and lymph, such as Busulfan and Fludarabine, thus the health of the patient is guaranteed.

Therapy of gene editing of autologous hematopoietic stem cells: a subject of hemoglobinopathy who is finally enrolled for treatment are determined by the companion diagnostic method developed in this application, then entering therapy process of gene-editing of autologous hematopoietic stem cells: 1. The subject will be administrated granulocyte colony stimulating factor (G-CSF) or/and plerixafor (an antagonist for removing chemokine receptor CXCR4) for mobilizing bone marrow, so that a large number of hematopoietic stem cells are produced by the bone marrow and released into the peripheral blood circulation system. 2. After the mobilization is completed, the production process of therapeutic cell products of gene editing is started; firstly, CD34-positive hematopoietic stem cells are obtained and cultured through CD34 magnetic bead labeling and sorting; secondly BCL11A erythroid enhancer site is genetically edited through introducing Cas9 and sgRNA by electroporation; finally, the cells are cryopreserved, entering the quality control and safety evaluation stage, and after reaching the dispatch standards, the cells are ready to start to be returned to the patient. 3. After the product is successfully prepared and meets the dispatch standards, the clinician will notify the subject to be hospitalized and receive pretreatment chemotherapy (administrating agents for clearing bone marrow e.g., busulfan and agents for clearing lymph e.g., cyclophosphamide and fludarabine). It should be noted that, different clinical institutions may adopt different pretreatment protocols. Some clinical institutions may only use agents for clearing bone marrow e.g., busulfan, while other clinical institutions may use agents for clearing bone marrow and agents for clearing lymph e.g., cyclophosphamide and fludarabine. After the pretreatment is completed, the genetically edited autologous hematopoietic stem cells are administrated and returned to the patient through a single intravenous injection at a dose of ≥2×10⁶ active CD34-positive cells/kg. 4. After the injection is completed, the subject will be followed up for at least 2 years to evaluate the indicators involving safety, tolerability and effectiveness, etc.

### INDUSTRIAL APPLICABILITY

According to the present application, the method of the present application has the following advantages. Firstly, the following phenomena are found in the present application for the first time: in different individuals, after gene editing of hematopoietic stem cells to down-regulate the expression of BCL11A, the increased levels and effect of γ-globin and fetal hemoglobin HbF are different; while in the same individual, after gene editing of hematopoietic stem cells to down-regulate the expression of BCL11A, the increased levels and effect of γ-globin and fetal hemoglobin HbF are consistent and stable; and this fills the gap in the prior art. Secondly, an effectiveness threshold of the increased level of fetal hemoglobin HbF after gene editing of BCL11A erythroid enhancer (i.e., the HbF level is increased by at least about 12% after gene editing) is set for the first time in this application, then after the patient receives treatment, the disease may be significantly improved, and it is possible to reduce the frequency of blood transfusions or get rid of blood transfusions. Thirdly, the companion diagnostic method developed in this application is simple to operate, and only a small amount of bone marrow or peripheral blood is needed to extract from a patient to be enrolled during the screening period (the patient screening period usually lasts 2-3 months), then isolating CD34-positive hematopoietic stem cells, performing gene editing of BCL11A erythroid enhancer and erythrocyte differentiation, and detecting the increased levels of γ-globin and fetal hemoglobin HbF; the entire evaluation process only needs 3-4 weeks to complete, thereby avoiding a risk of ineffective treatment method or insignificant effects for a patient caused by individual differences of the regulation of BCL11A gene on γ-globin and fetal hemoglobin HbF. Fourthly, the method developed in this application will be beneficial to stratifying and grading the patients in therapeutic protocol for treating hemoglobinopathy by gene editing technology, i.e., patients with higher increased levels of γ-globin and fetal hemoglobin HbF are at the priority level of treatment. Fifthly, this application proposes a novel therapeutic protocol combining the companion diagnosis and the therapy of gene-editing of autologous hematopoietic stem cells, thereby improving the safety and effectiveness of treating hemoglobinopathy by gene-editing.

## Claims

1. A method for treating hemoglobinopathy in an individual, which comprises:
a) an evaluation step, comprising: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells"); and
b) a treatment step, comprising: administering a second population of the modified CD34-positive HSPCs to the individual, wherein the modified CD34-positive HSPCs are derived from the individual and are modified to reduce BCL11A function ("modified TR cells").

2. A method for treating hemoglobinopathy in an individual, which comprises a treatment step comprising: administering a second population of the modified CD34-positive HSPCs to the individual, wherein the modified CD34-positive HSPCs are derived from the individual and are modified to reduce BCL11A function ("modified TR cells"), and
wherein the individual is selected for treatment based on the functional evaluation from an evaluation step which comprises: evaluating the ability of a first population of the modified CD34-positive hematopoietic stem cells/progenitor cells ("CD34-positive HSPCs") to produce a desired level of γ-globin or fetal hemoglobin (HbF) after differentiation, wherein the modified CD34-positive HSPCs of the first population are derived from the individual and are modified to reduce BCL11A function ("modified EV cells").

3. A method for determining whether an individual suffering from hemoglobinopathy is suitable or unsuitable for treating with a second population of the modified CD34-positive HSPCs ("modified TR cells") derived from the individual and modified to reduce BCL11A function, wherein the method comprises an evaluation step comprising: evaluating the ability of a first population of the modified CD34-positive HSPCs to produce a desired level of γ-globin or fetal hemoglobin (HbF) after differentiation, wherein the modified CD34-positive HSPCs of the first population are derived from the individual and are modified to reduce BCL11A function ("modified EV cells"), and wherein if the first population of the modified CD34-positive HSPCs produce a desired level of γ-globin or fetal hemoglobin (HbF), the individual is suitable for treatment; wherein if the first population of the modified CD34-positive HSPCs do not produce a desired level of γ-globin or fetal hemoglobin (HbF), the individual is not suitable for treating with the modified TR cells.

4. The method according to any one of claims 1-3, wherein the evaluation step comprises:
a) isolating CD34-positive HSPCs from the bone marrow or peripheral blood sample of the individual to obtain an isolated CD34-positive HSPCs population ("isolated EV cells");
b) modifying the isolated EV cells to obtain a first population of the modified CD34-positive HSPCs cells with reduced BCL11A function ("modified EV cells"); and
c) evaluating the ability of the modified EV cells to produce a desired level of γ-globin or fetal hemoglobin (HbF) after differentiation.

5. The method according to any one of claims 1-4, wherein the treatment step comprises:
a) mobilize the CD34-positive HSPCs in the bone marrow of the individual to increase the amount of CD34-positive HSPCs in the peripheral blood;
b) isolating CD34-positive HSPCs from the peripheral blood of the individual to obtain an isolated CD34-positive HSPCs population ("isolated TR cells"),
c) modifying the isolated TR cells to obtain a second population of the modified CD34-positive HSPCs with reduced BCL11A function ("modified TR cells"); and
d) administering an effective amount of the modified TR cells to the individual.

6. The method according to any one of claims 1-5:
1) the evaluation step comprises:
a) isolating CD34-positive HSPCs from the bone marrow or peripheral blood sample of the individual to obtain an isolated CD34-positive HSPCs population ("isolated EV cells");
b) modifying the isolated EV cells to obtain a first population of the modified CD34-positive HSPCs cells with reduced BCL11A function ("modified EV cells"); and
c) evaluating the ability of the modified EV cells to produce a desired level of γ-globin or fetal hemoglobin (HbF) after differentiation; and
2) the treatment step comprises:
a) mobilizing the CD34-positive HSPCs in the individual to the peripheral blood;
b) isolating CD34-positive HSPCs from the peripheral blood of the individual to obtain an isolated CD34-positive HSPCs population ("isolated TR cells"),
c) modifying the isolated TR cells to obtain a second population of the modified CD34-positive HSPCs with reduced BCL11A function ("modified TR cells"); and
d) administering an effective amount of the modified TR cells to the individual.

7. The method according to any one of claims 1-6, wherein the modified EV cells are modified by genetic modification.

8. The method according to any one of claims 4-6, wherein modifying the isolated EV cells comprises genetically modifying the isolated EV cells.

9. The method according to claim 7 or 8, wherein the isolated EV cells are genetically modified by a technology selected from the group consisting of: zinc finger nuclease (ZFN), transcription activator-like effector nuclease (TALEN), and clustered regularly interspaced short palindromic repeats (CRISPRs), RNA editing, RNA interference.

10. The method according to any one of claims 1-9, wherein the ability of the modified EV cells to produce a desired level of γ-globin or fetal hemoglobin (HbF) after differentiation is evaluated in the evaluation step comprising: 1) culturing the modified EV cells under conditions allowing differentiation to obtain an erythrocyte population; and 2) determining the level of γ-globulin or fetal hemoglobin (HbF) produced by the erythrocytes.

11. The method according to any one of claims 1-10, wherein the ability of the modified EV cells to produce a desired level of γ-globin or fetal hemoglobin (HbF) after differentiation is evaluated in the evaluation step comprising: determining the mRNA level of γ-globulin.

12. The method according to any one of claims 1-10, wherein the ability of the modified EV cells to produce a desired level of γ-globin or fetal hemoglobin (HbF) after differentiation is evaluated in the evaluation step comprising: determining the protein level of fetal hemoglobin (HbF).

13. The method according to any one of claims 1-12, wherein the individual has not undergone mobilization or pretreatment prior to the evaluation step.

14. The method according to any one of claims 1-13, wherein the evaluation step is repeated at least once prior to the treatment step.

15. The method according to any one of claims 1-14, wherein the modified TR cells are modified by genetic modification.

16. The method according to any one of claims 5-14, wherein modifying the isolated TR cells comprises genetically modifying the isolated TR cells.

17. The method according to claim 15 or 16, wherein the isolated TR cells are genetically modified by a technology selected from the group consisting of: zinc finger nuclease (ZFN), transcription activator-like effector nuclease (TALEN), clustered regularly interspaced short palindromic repeats (CRISPRs), RNA editing, and RNA interference.

18. The method according to any one of claims 5-17, wherein mobilizing CD34-positive HSPCs in the treatment step comprises: treating the individual with granulocyte colony stimulating factor (GCSF) and/or plerixafor.

19. The method according to any one of claims 1-18, wherein the treatment step further comprises: pretreating the individual prior to administering the modified TR cells.

20. The method according to claim 19, wherein the pretreatment comprises chemotherapy, monoclonal antibody therapy, or systemic radiation.

21. The method according to claim 20, wherein the pretreatment comprises chemotherapy.

22. The method according to claim 21, wherein the chemotherapy comprises administering to the individual one or more chemotherapeutic agents selected from the group consisting of: busulfan, cyclophosphamide, and fludarabine.

23. The method according to any one of claims 5-22, wherein the isolated TR cells are cultured for one or more days prior to modification.

24. The method according to any one of claims 5-23, wherein the modified TR cells are cultured for one or more days prior to being administered to the individual.

25. The method according to any one of claims 1-24, wherein the modified TR cells are stored under a freezing condition for at least 24 hours prior to administering the modified TR cells to the individual.

26. The method according to claim 25, wherein the modified TR cells are cultured for one or more days prior to being stored under a freezing condition.

27. The method according to any one of claims 1-26, wherein the hemoglobinopathy is a disease selected from the group consisting of: sickle cell disease, sickle cell trait, hemoglobin C disease, hemoglobin C trait, hemoglobin S/C disease, hemoglobin D disease, hemoglobin E disease, thalassemia, hemoglobin-related disorder with increased oxygen affinity, hemoglobin-related disorder with decreased oxygen affinity, unstable hemoglobin disease and methemoglobinemia.

28. The method according to claim 27, wherein the hemoglobinopathy is selected from the group consisting of: β-thalassemia and sickle cell anemia.

29. The method according to claim 28, wherein the hemoglobinopathy is β⁰ or β⁺ thalassemia.

30. The method according to any one of claims 1-29, wherein the individual is a human.

31. The method according to any one of claims 1-30, wherein the treatment step is performed immediately following the evaluation step.
